# EUROPEAN PATENT APPLICATION

(11) **EP 4 691 339 A1**
(43) Date of publication of application: **11.02.2026**
(21) Application number: 24779053.8
(22) Date of filing: 01.03.2024
(51) Int. Cl.: A61B 1/045, A61B 1/00, G01N 21/64, G02B 21/00

(54) **IMAGE GENERATION DEVICE, MEDICAL OBSERVATION SYSTEM, AND IMAGE GENERATION METHOD**

(30) Priority: 29.03.2023 JP 2023054243
(71) Applicant: Sony Olympus Medical Solutions Inc., Hachioji-shi, Tokyo 192-0904 (JP)
(72) Inventor: YAMAMOTO, Takahiro, Hachioji-shi, Tokyo 192-0904 (JP)
(74) Representative: D Young & Co LLP
(86) International application number: PCT/JP2024/007683
(87) International publication number: WO 2024/202915

(57) **Abstract**

Provided is a technology advantageous for devising and displaying a plurality of images related to an observation target.

An image generation device generates an output image on the basis of a first image that is an image obtained by imaging a first range of an observation target using a first imaging device and a second image that is an image obtained by imaging a second range different from the first range of the observation target using a second imaging device different in type from the first imaging device, and the output image is an image obtained by performing processing of replacing or deleting one or more pixel signals among pixel signals of the first image and the second image on the basis of the first range and the second range.

## Description

### TECHNICAL FIELD

The present disclosure relates to an image generation device, a medical observation system, and an image generation method.

### BACKGROUND ART

In the medical field, for example, an endoscope device is used to observe a subject in order to support surgery. An endoscope device can image a subject under irradiation with white light to obtain a captured image of the subject. On the other hand, fluorescence observation is effective for a subject tissue that is difficult to identify from a captured image using white light. For example, by irradiating a subject to which an agent capable of emitting fluorescence is administered with excitation light and observing fluorescence emitted from the subject, it is possible to visually grasp the characteristics and state of the subject tissue.

In particular, according to a superimposed image obtained by combining white light-captured image and fluorescence-captured image, various characteristics and states of the subject can be simultaneously visualized. For example, in a medical image processing device disclosed in Patent Document 1, an output image in which a first captured image (e.g., white light-captured image) and a second captured image (e.g., fluorescence image) are superimposed is generated.

### CITATION LIST

### PATENT DOCUMENT

Patent Document 1: Japanese Patent Application Laid-Open No. 2021-132695

### SUMMARY OF THE INVENTION

### PROBLEMS TO BE SOLVED BY THE INVENTION

A user such as a doctor observes a subject via an image of the subject displayed on a display device. In particular, in a case where a plurality of images (e.g., normal light-captured image captured and acquired under white light irradiation and fluorescence-captured image captured and acquired under excitation light irradiation) of different types related to the subject is displayed on the display device, it is required to devise how to show the plurality of images so as not to lead to erroneous diagnosis when performing surgery.

The present disclosure provides a technology advantageous for devising and displaying a plurality of images related to an observation target.

### SOLUTIONS TO PROBLEMS

One aspect of the present disclosure relates to an image generation device that generates an output image on the basis of a first image that is an image obtained by imaging a first range of an observation target using a first imaging device and a second image that is an image obtained by imaging a second range different from the first range of the observation target using a second imaging device different in type from the first imaging device, in which the output image is an image obtained by performing processing of replacing or deleting one or more pixel signals among pixel signals of the first image and the second image on the basis of the first range and the second range.

The type may be a physical size, an image size, or a model number of the imaging device.

A first output image that is the output image, or a second output image generated by adding one or more pixel signals to an image generated from at least some of the pixel signals of the second image may be output.

One of the first range and the second range may be wider than the other.

The first image region of the output image may be generated on the basis of the first image and the second image.

The output image may include a first image region and a second image region different from the first image region.

An image visually distinguishable and identifiable from an image allocated to the first image region may be allocated to the second image region.

The output image may be configured only of the first image region.

The output image may include a boundary emphasis image indicating a boundary between the first image region and the second image region.

The second range may be included in the first range.

The output image may be generated on the basis of the entire second image.

The image generation device may enlarge or reduce one or both of the first image and the second image such that the number of pixels of a common image region that is an image region of a part of the observation target commonly included in the first image and the second image is consistent between the first image and the second image, and may generate an image allocated to the first image region of the output image on the basis of an image of the common image region in the first image and an image of the common image region in the second image having the same number of pixels.

The image generation device may be connectable to one or a plurality of display devices on which an output image is displayed, and may generate and output an output image corresponding to each characteristic of one or a plurality of display devices.

The first image may be acquired by imaging the observation target irradiated with first light in a first wavelength band, and the second image may be acquired by imaging the observation target irradiated with second light in a wavelength band at least partially different from the first wavelength band.

The first light is visible light; the second light may be excitation light that excites a specific substance so as to emit fluorescence; the first image includes a captured image based on reflected light of the visible light from the observation target; and the second image may include a captured image based on the fluorescence from the observation target irradiated with the excitation light.

Another aspect of the present disclosure relates to a medical observation system including an imaging apparatus including a first imaging device and a second imaging device different in type from the first imaging device, and an image generation device that generates an output image, in which the image generation device generates the output image on the basis of a first image that is an image obtained by imaging a first range of an observation target using the first imaging device and a second image that is an image obtained by imaging a second range different from the first range of the observation target using the second imaging device, and the output image is an image obtained by performing processing of replacing or deleting one or more pixel signals among pixel signals of the first image and the second image on the basis of the first range and the second range.

The medical observation system may include a light source device that emits light with which the observation target is irradiated, and the imaging apparatus may image the observation target irradiated with the light and acquire the first image and the second image.

Another aspect of the present disclosure relates to an image generation method including a step of generating an output image on the basis of a first image that is an image obtained by imaging a first range of an observation target using a first imaging device and a second image that is an image obtained by imaging a second range different from the first range of the observation target using a second imaging device different in type from the first imaging device, in which the output image is an image obtained by performing processing of replacing or deleting one or more pixel signals among pixel signals of the first image and the second image on the basis of the first range and the second range.

The image generation method may further include a size adjustment processing step of adjusting a size of both or one of the first image and the second image, and a step of generating the output image on the basis of the first image and the second image after the size adjustment processing step.

The image generation method may further include: a step of extracting an image of a range in which the observation target is captured in the first image; a size adjustment processing step of adjusting a size of both or one of the first image and the second image; and a step of generating the output image on the basis of the extracted first image and the second image after the size adjustment processing step.

### BRIEF DESCRIPTION OF DRAWINGS

Fig. 1 is a diagram illustrating an example of a medical observation system.
Fig. 2 is a diagram illustrating a schematic configuration of an example of a light source device.
Fig. 3 is a diagram illustrating a schematic configuration of an example of an imaging system of a camera head including two imaging devices.
Fig. 4 is a diagram illustrating a schematic configuration of another example of the imaging system of a camera head including two imaging devices.
Fig. 5 is a diagram illustrating a schematic configuration of an example of an imaging system of a camera head including three imaging devices.
Fig. 6 is a diagram illustrating a schematic configuration of another example of the imaging system of a camera head including three imaging devices.
Fig. 7 is a block diagram illustrating a configuration example of a camera head and a control device.
Fig. 8 is a flowchart illustrating an example of a medical observation method performed by the medical observation system.
Fig. 9 illustrates an example of a physical size relationship between an imaging region (effective pixel region) of a first imaging device and an imaging region (effective pixel region) of a second imaging device.
Fig. 10 is a schematic diagram illustrating an example of a first image generated on the basis of an image signal from the first imaging device illustrated in Fig. 9.
Fig. 11 is a diagram illustrating an example of generation processing of a first image, a second image, and a superimposed image in a first embodiment.
Fig. 12 illustrates an example of a first output image generated mainly from a first image (normal light-captured image) which is an image captured by the first imaging device.
Fig. 13 illustrates an example of a second output image mainly generated from a second image (fluorescence-captured image) which is an image captured by the second imaging device.
Fig. 14 is a diagram illustrating an example of output image generation processing in the first embodiment.
Fig. 15A is a flowchart illustrating an example of superimposed output image generation processing.
Fig. 15B is a flowchart illustrating an example of superimposed output image generation processing.
Fig. 15C is a flowchart illustrating an example of superimposed output image generation processing.
Fig. 16 is a diagram illustrating an example of generation processing of a first image, a second image, and a superimposed image in a second embodiment.
Fig. 17 is a diagram illustrating an example of output image generation processing in the second embodiment.
Fig. 18 is a conceptual diagram illustrating an example of a medical observation system configured as an operative field illumination observation device in which a ring light (open field illumination device for observing biological tissue) is connected to a light source device.
Fig. 19 is a diagram illustrating an example of a medical observation system configured as a microscope system.

### MODE FOR CARRYING OUT THE INVENTION

Hereinafter, embodiments of the present disclosure will be described with reference to the drawings. The same or corresponding elements are denoted by the same reference numerals, and the detailed description thereof will be omitted as appropriate. In addition, the terms "first", "second", and the like do not define a specific order, and do not represent importance, and are used merely for the purpose of distinction, unless otherwise noted.

Hereinafter, a case where the present disclosure technology is applied to an endoscope device will be exemplified. Note, however, that the application target of the present disclosure technology is not limited, and the present disclosure technology can also be applied to a system, a device, and a method for medical observation other than the endoscope device. The term "medical" as used herein is interpreted in a broad sense, and is a concept that can include not only treatment of diseases and injuries but also various actions for the purpose of maintaining, recovering, or promoting health, and an action for the main purpose of research is also included in the concept of "medical".

### [Medical observation system]

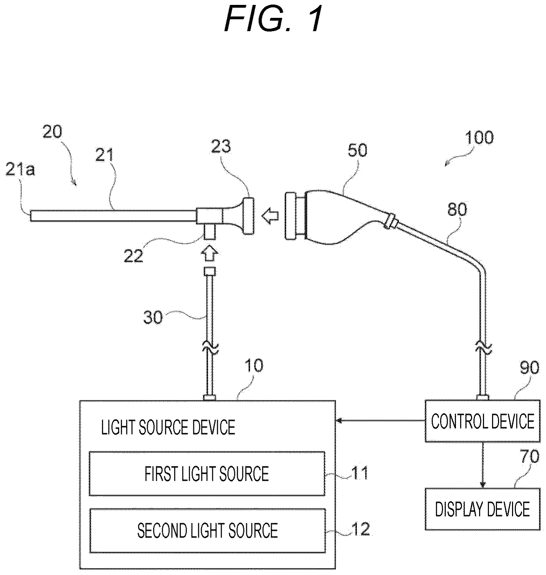

Fig. 1 is a diagram illustrating an example of a medical observation system 100.

A medical observation system 100 illustrated in Fig. 1 is configured as an endoscope device (endoscope system) for observing light (i.e., observation light) from a subject to be observed via a captured image. Note, however, that the medical observation system 100 of this example also enables direct visual recognition of observation light with the naked eye without passing through the captured image as described later.

The medical observation system 100 illustrated in Fig. 1 includes a light source device 10, an insertion device 20 (endoscope main body), a light guide 30, a camera head (imaging unit) 50, a display device 70, a transmission cable 80, and a control device 90.

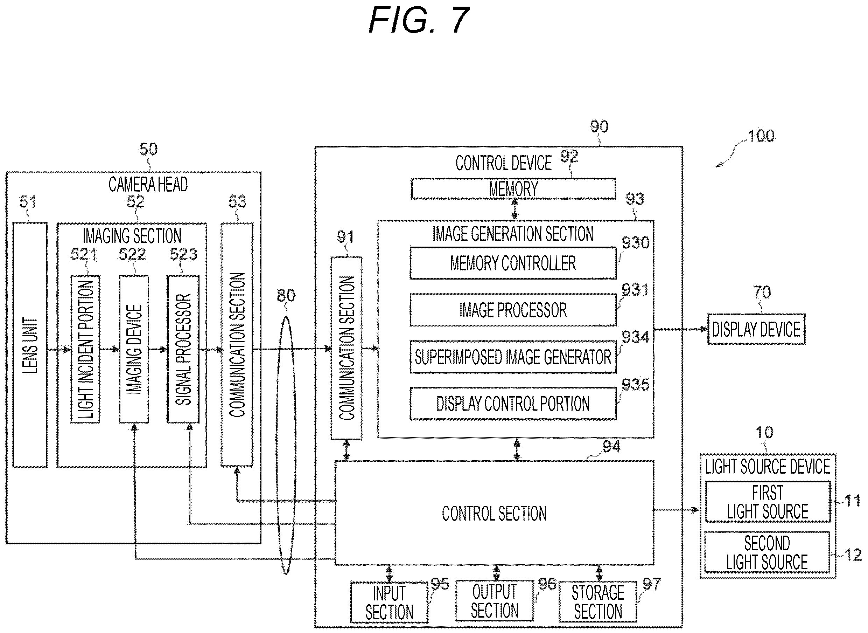

The light source device 10 is a device that emits light applied to a subject to be observed, and emits light under the control of a control device 90 (particularly "control unit" described later (see Fig. 7)). The light source device 10 can emit a plurality of types of light having different wavelength bands, and can emit one or both of visible light (white light or the like) and invisible light (infrared light, ultraviolet light or the like), for example.

The light source device 10 illustrated in Fig. 1 includes a first light source 11 that emits broadband light and a second light source 12 that emits narrowband light.

The broadband light emitted from the first light source 11 includes, as a main light component, light in a relatively wide wavelength band (i.e., first wavelength band) compared to the narrowband light. The first wavelength band of the broadband light may be a continuous single wavelength band or may include a plurality of discrete wavelength bands. The wavelength band of the broadband light is not limited, and as an example, the first light source 11 may be visible light (e.g., white light).

On the other hand, the narrowband light emitted from the second light source 12 includes, as a main light component, light in a relatively narrow wavelength band as compared to the broadband light. The narrowband light emitted from the second light source can act as light that excites a specific substance that emits fluorescence, and may be partially or entirely included in the first wavelength band that is the wavelength band of the broadband light emitted from the first light source, or may not be included in the first wavelength band. The fluorescence emitted from the substance excited by the narrowband light from the second light source has a bandwidth narrower than the first wavelength band, but may have an arbitrary wavelength band, and may be visible light or invisible light.

The light source device 10 is connected to the insertion device 20 via the light guide 30, and light emitted from the light source device 10 is transmitted to the insertion device 20 via the light guide 30. The light guide 30 of this example is detachably connected to the light source device 10 and the insertion device 20.

The insertion device 20 includes an insertion portion 21, and an optical connection portion 22 and an imaging connection portion 23 provided on the proximal end side of the insertion portion 21. The insertion device 20 illustrated in Fig. 1 is configured as a rigid endoscope, and the insertion portion 21 has a rigid and elongated shape. Note, however, that the insertion device 20 can have any structure, and may be configured as, for example, a flexible endoscope having a flexible insertion portion 21.

A light transmission portion (light guide) and an objective lens are provided on an end surface of a distal end portion 21a of the insertion portion 21 located on the side opposite to the proximal end side. Light transmitted from the light source device 10 to the insertion device 20 via the light guide 30 is emitted from the light transmission portion on the end surface of the distal end portion 21a of the insertion portion 21 toward the observation target. Then, light from the observation target enters the objective lens, and is guided to the imaging connection portion 23 through the inside of the insertion portion 21. In this manner, the entire light from the observation target guided to the imaging connection portion 23 via the objective lens and the inside of the insertion portion 21 is referred to as observation light, and, for example, reflected light from the observation target and fluorescence emitted from the observation target can be included in observation light.

The imaging connection portion 23 is detachably connected to a connection portion of the camera head 50. Observation light transmitted through the objective lens enters the camera head 50 through the imaging connection portion 23 and is received by the camera head 50.

Note that the imaging connection portion 23 can also function as an eyepiece portion. That is, in a state where the imaging connection portion 23 is detached from the camera head 50, the operator of the insertion device 20 can also directly view the observation light via the imaging connection portion 23.

The camera head 50 is an imaging apparatus that is detachably connected to the insertion device 20 and receives observation light transmitted via the insertion device 20, and images an observation target irradiated with light emitted by the light source device 10 to acquire an image (first image and second image to be described later). The camera head 50 is connected to the control device 90 via the transmission cable 80.

The transmission cable 80 can transmit various signals (e.g., image signal, control signal, synchronization signal, and clock signal) and power between the camera head 50 and the control device 90. The camera head 50 outputs an image signal corresponding to the received observation light, and the image signal is transmitted from the camera head 50 to the control device 90 via the transmission cable 80.

The signal transmission method of the transmission cable 80 is not limited, and various signals can be transmitted via the transmission cable 80 as an electric signal or an optical signal. In place of the wired signal transmission method via the transmission cable 80, various signals may be transmitted between the camera head 50 and the control device 90 by a wireless signal transmission method (e.g., wireless local area network (LAN), Bluetooth (registered trademark), infrared communication, and the like).

The control device 90 is connected to the camera head 50, the light source device 10, and the display device 70 in a wired or wireless manner, and integrally controls the camera head 50, the light source device 10, and the display device 70. The control device 90 controls light emission of the light source device 10 as described later, generates an image from an image signal transmitted from the camera head 50, and causes the display device 70 to display the image. Furthermore, the control device 90 can also control the insertion device 20 connected to the camera head 50 via the camera head 50.

Note that the control device 90 is provided separately from the light source device 10, the camera head 50, and the display device 70 in the example illustrated in Fig. 1, but may be provided integrally with the light source device 10, the camera head 50, and/or the display device 70.

The display device 70 includes an arbitrarily configured display (e.g., liquid crystal display or organic electro-luminescence (EL) display), and displays an image on the display under the control of the control device 90.

Although the display device 70 is illustrated as a single block in Fig. 1, one or a plurality of display devices 70 can be connected to the control device 90 (image generation device). In a case where the plurality of display devices 70 is connected to the control device 90, the same output image may be synchronously displayed on the plurality of display devices 70, or different output images may be synchronously displayed on the plurality of display devices 70. For example, while an output image in which the entire observation target is captured in a bird's-eye view is displayed on a certain display device 70, an output image in which the entire or a part of the observation target is enlarged and captured may be displayed on another display device 70.

### [Light source device]

Next, a device configuration example of the above-described light source device 10 will be described.

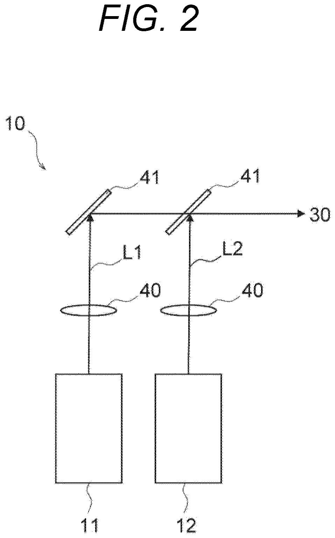

Fig. 2 is a diagram illustrating a schematic configuration of an example of the light source device 10. In the light source device 10 illustrated in Fig. 2, a lens optical system 40 and a mirror optical system 41 are provided for each of the first light source 11 and the second light source 12.

The lens optical system 40 includes a collimator lens that collimates incident light. The mirror optical system 41 reflects light incident through the lens optical system 40 and transmits light incident through another mirror optical system 41.

A broadband light L1 emitted from the first light source 11 is collimated by the corresponding lens optical system 40, then reflected by the corresponding mirror optical system 41, transmitted through the mirror optical system 41 associated with the second light source 12, and incident on the light guide 30. A narrowband light L2 emitted from the second light source 12 is collimated by the corresponding lens optical system 40, then reflected by the corresponding mirror optical system 41, and incident on the light guide 30.

In particular, in the light source device 10 of this example, the broadband light L1 and the narrowband light L2 emitted from the first light source 11 and the second light source 12 are reflected by the corresponding mirror optical system 41, and then incident on the light guide 30 through a common optical path. Therefore, in a case where the first light source 11 and the second light source 12 simultaneously emit light, the broadband light L1 and the narrowband light L2 are incident on the light guide 30, whereby the observation target is irradiated with the broadband light L1 and the narrowband light L2.

Note that the light source device 10 illustrated in Fig. 2 is merely an example, and the light source device 10 can have another arbitrary configuration. For example, the light source device 10 may include another additional light source that emits light in a wavelength band different from the broadband light L1 from the first light source 11 and the narrowband light L2 from the second light source 12. As an example, the additional light source may emit narrowband light that excites a specific substance different from the substance excited by the light from the second light source 12 and includes light in a wavelength band narrower than that of the broadband light as a main light component. The fluorescence emitted from the substance excited by the narrowband light from the additional light source also has a bandwidth narrower than the first wavelength band, but may have any wavelength band, and may be visible light or invisible light.

Moreover, specific device configurations of the first light source 11 and the second light source 12 are not limited, and each of the first light source 11 and the second light source 12 may have a single light emitting device or a plurality of light emitting devices. In addition, the wavelength bands of the broadband light L1 and the narrowband light L2 emitted by the first light source 11 and the second light source 12 are not limited.

For example, the first light source 11 may emit visible light as the broadband light L1, or may emit light in a wavelength band corresponding to a color gamut displayable by the display device 70. Visible light is light perceptible to healthy human eyes and may have a wavelength band of 380 nm to 780 nm as an example, with an upper wavelength limit of 760 nm to 830 nm and a lower wavelength limit of 360 nm to 400 nm. Note that light other than visible light is referred to as invisible light, and a wavelength band other than the visible light wavelength band is referred to as an invisible light wavelength band.

Therefore, the first light source 11 may emit white light as the broadband light L1, and may include a light-emitting diode (LED), a xenon lamp, or any other device. The first light source 11 may include a white LED that is a monochromatic light emitting device, or may include a plurality of colored LEDs (e.g., red LED, green LED, and blue LED) that emit light of different colors. The first light source 11 may include a white LED and a plurality of colored LEDs that emit colored light other than white light.

Furthermore, the light emitted from the first light source 11 may include at least a part of light in the visible light wavelength band, and, for example, may include light corresponding to the color gamut of the standard specification of the display device 70, or may include light in a specific wavelength band (e.g., violet light or green light).

The second light source 12 may emit, as the narrowband light L2, light that excites a substance that emits fluorescence in a wavelength band included in the wavelength band (first wavelength band) of the broadband light L1, or may emit light that excites a substance that emits fluorescence in a wavelength band not included in the first wavelength band.

Therefore, when the broadband light L1 from the first light source 11 is visible light, the substance excited by the narrowband light L2 may emit fluorescence in the visible light wavelength band or may emit fluorescence (e.g., infrared light or ultraviolet light) in the invisible light wavelength band.

The second light source 12 may include, for example, a laser light source, an LED light source, a xenon lamp, or any other light emitting device. As an example, the second light source 12 may be configured by combining such a light emitting device (e.g., xenon lamp) and a filter (band pass filter) that transmits narrowband light of a desired wavelength band among light emitted from the light emitting device.

Note that the substance excited by the light (e.g., narrowband light L2) emitted from the light source device 10 may be an agent or a fluorescent dye applied to the observation target, or may be a fluorescent substance constituting the observation target itself.

Examples of such an agent that can be applied to the observation target include 5-ALA (PP-IX), ADS780WS, ADS830WS, aggregation-induced emission dots allophycocyanin (APC), boron-dipyrromethane (BODIPY), CLR 1502, Flavins, fluorescamine, Fluorescein, fluoro-gold, green fluorescence protein, indocyanine green (ICG), IRDye 78, IR-PEG nanoparticles, Isothiocyanate, rose Bengal, and trypan blue.

Examples of the fluorescent dye that can be applied to the observation target include coumarine, Cy3, DyLight547, GE3126, metal nanoclusters, oxacarbocyanine, Rhodamine, Riboflavin, fluorescein, AlexaFluor660, AlexaFluor680, AlexaFluor700, Cy5, Cy5.5, Dy677, Dy682, Dy752, DyLight647, HiLyte Fluor 647, HiLyte Fluor 680, IRDye 700DX, methylene blue, Porphyrins, Porphysomes, VivoTag-680, VivoTag-S680, AlexaFluor750, AlexaFluor790, carbocyanine, conjugated copolymers, CW800-CA, Cy7, Cy7.5, cyanine dyes, Dy780, HiLyte Fluor 750, Indocarbocyanine, IR-786, IRDye 800CW, IRDye 800RS, IRDye 800BK, Nervelight^{™}, OTL-38, Polymethine, VivoTag-S750, and Xanthene.

Examples of the fluorescent substance derived from the observation target constituting the observation target itself include collagen, elastin, and NADH.

### [Camera head]

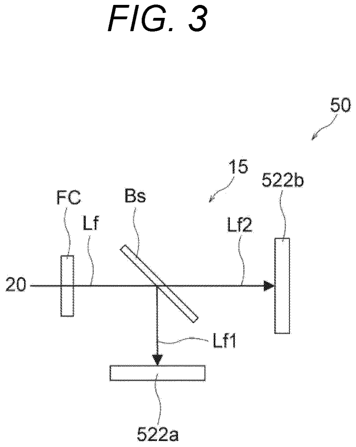

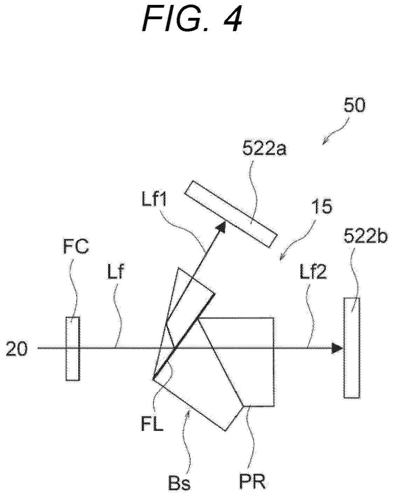

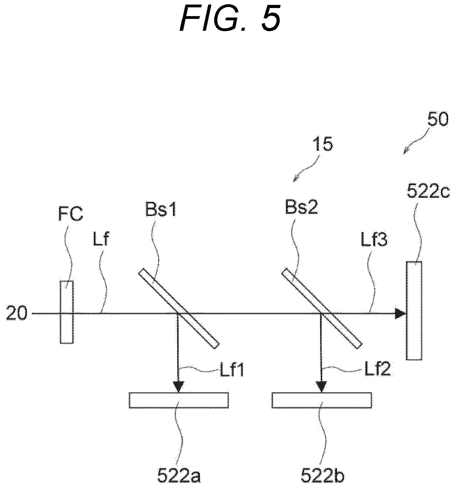

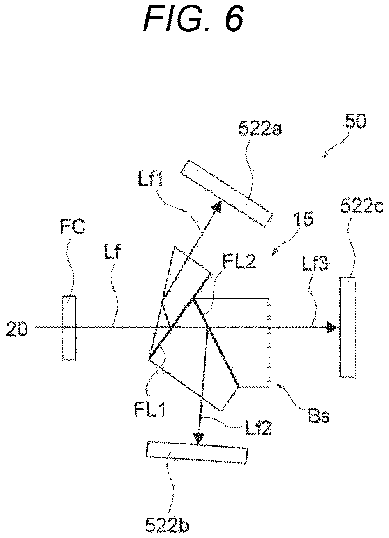

Next, a configuration example of an imaging system of the above-described camera head 50 will be described. Hereinafter, a typical example of a two-plate type imaging module (see Figs. 3 and 4) that performs imaging using two imaging devices and a typical example of a so-called three-plate type imaging module (see Figs. 5 and 6) that performs imaging using three imaging devices will be described.

Fig. 3 is a diagram illustrating a schematic configuration of an example of an imaging system of a camera head 50 including two imaging devices 522a and 522b.

The camera head 50 illustrated in Fig. 3 includes an excitation light cut filter FC, a branching optical system Bs, a first imaging device 522a, and a second imaging device 522b.

Observation light Lf transmitted through the insertion device 20 is incident on the branching optical system Bs after light in a predetermined wavelength band is cut by the excitation light cut filter FC.

The wavelength band of the light cut by the excitation light cut filter FC includes the wavelength band of the excitation light that may be emitted to the observation target. For example, when there is a possibility that the narrowband light from the second light source 12 is emitted to the observation target as the excitation light, the excitation light cut filter FC partially, substantially, or completely suppresses the light in the wavelength band of the narrowband light. The excitation light cut filter FC can be configured by a known wavelength selection filter or the like, and prevents reflected light of the excitation light with which the observation target is irradiated from being received by the imaging device (in this example, first imaging device 522a and second imaging device 522b).

Note that although Fig. 3 illustrates the excitation light cut filter FC as a single unit, the excitation light cut filter FC may include a single filter or a plurality of filters. For example, when there is a possibility that the observation target is irradiated with a plurality of types of excitation light having different wavelength bands, the excitation light cut filter FC may be a single filter that partially, substantially, or completely suppresses light in the wavelength bands of these excitation lights, or may include a plurality of filters that partially, substantially, or completely suppresses light in the wavelength bands of the respective excitation lights.

Furthermore, the excitation light cut filter FC is not limited to the position illustrated in Fig. 3, and can be installed at an arbitrary position on the optical path of the observation light Lf (including light flux after separation) from the observation target to the imaging device (first imaging device 522a and second imaging device 522b in this example). That is, the excitation light cut filter FC may be provided in the insertion device 20, and, for example, may be provided on the upstream side or the downstream side of the objective lens of the end surface of the distal end portion 21a of the insertion portion 21 in the traveling direction of the observation light Lf. The excitation light cut filter FC may be provided separately from the medical observation system 100.

Note that the excitation light cut filter FC may be omitted. When the influence of the reflected light of the excitation light on the captured image is sufficiently small, the system configuration may be simplified by not providing the excitation light cut filter FC.

The branching optical system Bs is an optical element 15 that separates the observation light Lf from the observation target into a first light flux Lf1 and a second light flux Lf2 (plurality of light fluxes), guides the first light flux Lf1 to the first imaging device 522a, and guides the second light flux Lf2 to the second imaging device 522b. The branching optical system Bs illustrated in Fig. 3 reflects the first light flux Lf1 toward the first imaging device 522a and transmits the second light flux Lf2 to guide the second light flux Lf2 to the second imaging device 522b.

The branching optical system Bs can be configured on the basis of, for example, a combination of a dichroic mirror and a wavelength selection filter, but a specific configuration of the branching optical system Bs is not limited. The branching optical system Bs may have an optical device that separates (disperses) incident light into a plurality of light fluxes on the basis of the wavelength, and, for example, light in the visible light wavelength band and light in the invisible light wavelength band in incident light may be separated into separate light fluxes. In addition, the branching optical system Bs may include an optical device that separates incident light into a plurality of light fluxes not on the basis of the wavelength, and, for example, the incident light may be separated into a plurality of light fluxes having the same wavelength characteristic.

Note that in a case where the branching optical system Bs separates the incident light into a plurality of light fluxes on the basis of the wavelength, each light flux after the separation may include light of a wavelength band (i.e., unintended wavelength band) different from a wavelength band of light intended to be included as a main light component in each light flux. In the example illustrated in Fig. 3, when the branching optical system Bs separates the observation light Lf into the first light flux Lf1 in the visible light wavelength band and the second light flux Lf2 in the invisible light wavelength band, the second light flux Lf2 may include visible light corresponding to about 3% to 20% of the light amount in the visible light wavelength band in the observation light Lf.

Note that in a case where the light flux after separation includes light of an unintended wavelength band (also referred to herein as "leakage light"), the optical element 15 may include a filter that partially, substantially, or completely removes the leakage light from the light flux. For example, in a case where light included in the separated light flux is weak fluorescence, if the light flux includes leakage light, there is a possibility that fluorescence that is the original light to be received is not appropriately received by the corresponding imaging device (e.g., second imaging device 522b). In such a case, the leakage light is partially, substantially, or completely removed from the light flux by the filter, whereby the fluorescent light to be received is more appropriately received by the corresponding imaging device.

In addition, in a case where the branching optical system Bs separates incident light into a plurality of light fluxes regardless of wavelength, the plurality of light fluxes after the separation may have substantially equal light amounts or unequal light amounts. For example, in the example illustrated in Fig. 3, each of the first light flux Lf1 and the second light flux Lf2 separated by the branching optical system Bs may have a light amount of about 50% of the light amount of the observation light Lf, or may have different light amounts (e.g., light amount difference of about ±10% between first light flux Lf1 and second light flux Lf2). For example, the branching optical system Bs may be designed such that the light amount difference between the plurality of light fluxes is determined on the basis of the difference in sensitivity between the imaging devices that receive the plurality of light fluxes after separation.

As an example, in a case where the ICG applied to the observation target is excited, the branching optical system Bs may include a wavelength selection filter that reflects light on a shorter wavelength side than the vicinity of 820 nm to 870 nm, which is the fluorescence wavelength of the ICG, while transmitting light in another wavelength band.

Fig. 4 is a diagram illustrating a schematic configuration of another example of the imaging system of the camera head 50 including the two imaging devices 522a and 522b.

The branching optical system Bs included in the optical element 15 illustrated in Fig. 4 includes a color separation prism PR based on a combination of a plurality of (three) prisms and a wavelength selection filter FL. The wavelength selection filter FL is provided on a joint surface between a first prism located most upstream among the color separation prisms PR and a second prism adjacent to the first prism.

Other configurations are similar to those of the camera head 50 illustrated in Fig. 3 described above.

Part of the observation light Lf (first light flux Lf1) incident on the optical element 15 of this example is reflected by the joint surface between the first prism and the second prism of the color separation prism PR, and then further reflected to be guided to the first imaging device 522a. On the other hand, at least a part (second light flux Lf2) of the rest of the of the observation light Lf is transmitted through the optical element 15 and guided to the second imaging device 522b. Note that by providing an anti-reflection coating (AR coating) on the joint surface between the second prism and a third prism of the color separation prism PR, reflection of light on the joint surface can be effectively suppressed. Further, the color separation prism PR is not limited to the example illustrated in Fig. 4, and may have any configuration. For example, a two-plate prism formed by combining two prisms (first prism and second prism) may be used as the color separation prism PR.

The camera head illustrated in Figs. 3 and 4 described above is a typical example of a two-plate type imaging module that performs imaging using two imaging devices, but the camera head 50 may perform imaging using three or more imaging devices.

Fig. 5 is a diagram illustrating a schematic configuration of an example of an imaging system of the camera head 50 including three imaging devices 522a, 522b, and 522c.

The camera head 50 illustrated in Fig. 5 has a configuration similar to that of the camera head 50 illustrated in Fig. 3, but a first branching optical system Bs1 and a second branching optical system Bs2 are provided as the optical element 15, and the observation light Lf is separated into the first light flux Lf1 to a third light flux Lf3.

That is, the observation light Lf having passed through the excitation light cut filter FC is incident on the first branching optical system Bs1. The first branching optical system Bs1 reflects the first light flux Lf1 toward the first imaging device 522a while transmitting the rest of the observation light Lf. The observation light Lf transmitted through the first branching optical system Bs1 is incident on the second branching optical system Bs2. The second branching optical system Bs2 reflects the second light flux Lf2 toward the second imaging device 522b while transmitting the third light flux Lf3, which is the rest of the observation light Lf, to guide the third light flux Lf3 to the third imaging device 522c.

The first branching optical system Bs1 and the second branching optical system Bs2 can be configured on the basis of, for example, a combination of a dichroic mirror and a wavelength selection filter, but specific configurations of the first branching optical system Bs1 and the second branching optical system Bs2 are not limited.

Fig. 6 is a diagram illustrating a schematic configuration of another example of the imaging system of the camera head 50 including the three imaging devices 522a, 522b, and 522c.

The optical element 15 illustrated in Fig. 6 has a configuration similar to that of the optical element 15 illustrated in Fig. 4, but includes the color separation prism PR, a first wavelength selection filter FL1, and a second wavelength selection filter FL2 as the branching optical system Bs. The first wavelength selection filter FL1 is provided on a joint surface between the first prism located most upstream among the color separation prisms PR and the second prism adjacent to the first prism. The second wavelength selection filter FL2 is provided on a joint surface between the second prism and the third prism adjacent to the second prism.

A part of the observation light Lf incident on the optical element 15 of this example is reflected by the joint surface between the first prism and the second prism of the color separation prism PR, is further reflected thereafter, and is emitted from the optical element 15 toward the first imaging device 522a as the first light flux Lf1. On the other hand, the rest of the observation light Lf is transmitted through the joint surface between the first prism and the second prism. Then, a part of the observation light Lf is reflected by the joint surface between the second prism and the third prism, and then is emitted from the optical element 15 toward the second imaging device 522b as the second light flux Lf2. On the other hand, the rest of the observation light Lf is transmitted through the joint surface between the second prism and the third prism, and then is emitted from the optical element 15 toward the third imaging device 522c as the third light flux Lf.

As described above, by using a dichroic mirror and a plurality of prisms as the optical element 15, the observation light Lf can be separated into a plurality of light fluxes. Then, by appropriately selecting the optical characteristics such as the transmission wavelength band of the wavelength selection filter FL, light in a desired wavelength band can be included in each of the plurality of light fluxes separated from the observation light Lf.

In particular, the two-plate type imaging module (see Figs. 3 and 4) is advantageous for reducing size and cost of the structure of the camera head 50. On the other hand, according to the three-plate type imaging module (see Figs. 5 and 6), three types of light can be simultaneously received by the three imaging devices (first imaging device 522a to third imaging device 522c).

### [Functional configuration of medical observation system]

Next, a functional configuration example of the above-described medical observation system 100 will be described.

Fig. 7 is a block diagram illustrating a configuration example of the camera head 50 and the control device 90.

The camera head 50 includes a lens unit 51, an imaging section 52, and a communication section 53.

The lens unit 51 includes one or a plurality of lenses, collects observation light transmitted via the insertion device 20 (see Fig. 1), and guides the observation light to the imaging section 52.

The imaging section 52 receives the observation light transmitted via the lens unit 51 and outputs a corresponding image signal. The imaging section 52 illustrated in Fig. 7 includes a light incident portion 521, an imaging device 522, and a signal processor 523.

The imaging device 522 is a photoelectric conversion element that receives observation light transmitted via the light incident portion 521 and generates an image signal under the control of the control device 90 (particularly control section 94 to be described later). The imaging device 522 is a charge coupled device (CCD) image sensor or a complementary metal oxide semiconductor (CMOS) image sensor.

Exposure to the imaging device 522 can be controlled by an arbitrary shutter method, and either a mechanical shutter or an electronic shutter may be used. In each of the embodiments described later, a rolling shutter method is used, but another shutter method (e.g., global shutter method) may be used.

In a case where a plurality of imaging devices 522 is provided, two or more imaging devices 522 having different characteristics (e.g., sensitivity, resolution, pixel size, number of pixels, overall size, color filter, and/or the like) may be provided. Alternatively, two or more imaging devices 522 having the same characteristics may be provided.

The light incident portion 521 is an optical system device that guides observation light from the lens unit 51 to the light receiving surface of the imaging device 522. For example, in a case where a plurality of imaging devices 522 is provided, the light incident portion 521 includes the above-described optical element 15 (see Figs. 3 to 6) that separates the observation light into a plurality of light fluxes and guides the plurality of light fluxes to the plurality of imaging devices 522.

The signal processor 523 performs signal processing (e.g., auto gain control (AGC) processing or analog-to-digital (AD) conversion processing) on the image signal generated by the imaging device 522 under the control of the control device 90 (control section 94).

The communication section 53 communicates with the control device 90 (particularly communication section 91) via the transmission cable 80 under the control of the control device 90 (in particular, control section 94). The image signal (digital signal) output from the imaging section 52 is transmitted from the communication section 53 to the control device 90 (particularly communication section 91) via the transmission cable 80. The communication standard between the communication section 53 of the camera head 50 and the communication section 91 of the control device 90 is not limited, and the communication section 53 and the communication section 91 may be configured as high-speed serial interfaces.

The control device 90 includes the communication section 91, a memory 92, an image generation section 93, the control section 94, an input section 95, an output section 96, and a storage section 97.

The communication section 91 transmits the image signal transmitted from the camera head 50 via the transmission cable 80 to the image generation section 93 under the control of the control section 94.

The image generation section 93 performs various types of image processing under the control of the control section 94, and generates an image on the basis of an image signal from the imaging section 52. The image generation section 93 illustrated in Fig. 7 includes a memory controller 930, an image processor 931, a superimposed image generator 934, and a display control portion 935.

The memory controller 930 writes and reads data to and from the memory 92. For example, the memory controller 930 writes an image signal transmitted from the camera head 50 via the communication section 91 into the memory 92 as image data. Then, the memory controller 930 reads image data from the memory 92 as necessary, and provides the image data to the image processor 931, the superimposed image generator 934, and/or the display control portion 935.

Note that the memory 92 can be configured by, for example, a volatile memory or a nonvolatile memory, and can be used as a device capable of temporarily storing various data. Data that can be stored in the memory 92 is not limited. Therefore, the memory controller 930 may write image data and other data transmitted from the image processor 931, the superimposed image generator 934, and/or the display control portion 935 in the memory 92 or read them from the memory 92.

The image processor 931 performs image processing on the image signal transmitted from the camera head 50 and generates a corresponding image (captured image). The image processor 931 can perform arbitrary image processing. The image processor 931 may perform, for example, one or more processing of AGC processing, optical black subtraction processing, white balance adjustment processing, demosaic processing, gradation correction processing, image (video) signal level adjustment processing, color correction processing, gamma correction processing, and fluorescence image signal processing.

The specific configuration of the image processor 931 is not limited, and the image processor 931 may include a single processor regardless of the number of imaging devices 522, or may include a plurality of processors corresponding to each of the plurality of imaging devices 522. In a case where a plurality of imaging devices 522 is assigned to one processor of the image processor 931, a plurality of corresponding images may be generated by sequentially processing a plurality of image signals from the plurality of imaging devices 522 by the one processor.

Furthermore, for example, in a case where two imaging devices 522 are provided, the image processor 931 may include a first processor that generates an image on the basis of an image signal from one imaging device 522 and a second processor that generates an image on the basis of an image signal from the other imaging device 522. In a case where the image processor 931 includes a plurality of processors exclusively assigned to each of the plurality of imaging devices 522, it is possible to simultaneously perform image generation processing based on image signals from the plurality of imaging devices 522 in parallel.

The superimposed image generator 934 combines a plurality of images to generate a superimposed image. The types and the specific composition method of the plurality of images as the basis of the superimposed image generated by the superimposed image generator 934 are not limited.

For example, the superimposed image may be generated by combining a captured image based on reflected light from the observation target irradiated with white light (broadband light) and a captured image based on fluorescence from the observation target irradiated with an excitation light (narrowband light). Alternatively, the superimposed image may be generated by combining captured images based on a plurality of fluorescences having different wavelength bands from the common observation target.

The superimposed image generator 934 may perform combining processing on the entire region of each of the plurality of images as the basis of the superimposed image, or may perform combining processing only on a partial region thereof. The plurality of images as the basis of the superimposed image may include images of two or more imaging devices 522 having different angles of view. In this case, the superimposed image generator 934 may perform the composition processing for generating the superimposed image after adjusting the angle of view of the images of the two or more imaging devices using an arbitrary image processing technology such as digital zoom.

The superimposed image generator 934 may automatically generate a superimposed image of a plurality of mutually related images under the control of the control section 94, or may generate a superimposed image using a plurality of images designated by the user as original images. The user may specify a plurality of original images via the input section 95, and the control section 94 may control the superimposed image generator 934 to generate a superimposed image from the plurality of original images specified via the input section 95.

The display control portion 935 generates an output image under the control of the control section 94. The display control portion 935 can generate an output image on the basis of the captured image generated by the image processor 931 and/or the superimposed image generated by the superimposed image generator 934. The output image generated by the display control portion 935 may include only a single image of the captured image and the superimposed image, or may include a plurality of images.

In a case where the output image includes a plurality of images, an arrangement mode of the plurality of images in the output image is not limited. Typically, the output image can take a picture-in-picture (PiP) format in which a certain image is superimposed on another image or a picture-by-picture (PbP) format in which a plurality of images is arranged. Therefore, the display control portion 935 may generate the output image in the PiP format in which one or a plurality of related captured images is reduced and arranged in a partial region of the superimposed image.

In addition, the output image generated by the display control portion 935 may be a still image or a moving image (video).

The output image generated by the display control portion 935 is transmitted to the display device 70 and displayed on the display of the display device 70. In a case where a plurality of display devices 70 is connected to the control device 90, the display control portion 935 generates an output image corresponding to the characteristic of each of the plurality of display devices 70 and outputs the output image to each of the plurality of display devices 70. For example, in a case where two or more display devices 70 having different screen resolutions (i.e., display total pixels) are connected to the control device 90, the display control portion 935 may generate a plurality of types of output images having the number of pixels corresponding to the screen resolutions of the two or more display devices 70, respectively. As a result, an output image having an optimized size is displayed on each of the two or more display devices 70.

The display control portion 935 may automatically generate the output image under the control of the control section 94, or may generate the output image according to the designation of the user. The user may specify one or a plurality of images to be included in the output image via the input section 95, and the control section 94 may control the display control portion 935 to generate the output image from the one or a plurality of images specified via the input section 95 and transmit the output image to the display device 70. As a result, the user can determine an output image to be displayed on the display device 70 on the basis of his/her own intention, and can switch the output image as necessary.

The control section 94 controls the light source device 10, the camera head 50, and the display device 70, and also controls each section (communication section 91, image generation section 93, input section 95, output section 96, storage section 97, and the like) of the control device 90. The target of the control performed by the control section 94 is not limited. For example, in each embodiment described later, light emission in the light source device 10, reading of image data from the imaging device 522, and image processing in the image generation section 93 are appropriately performed under the control of the control section 94.

For example, in a case where the sensitivity of the imaging device 522 is insufficient, the control section 94 may control the imaging device 522 to reduce the frame rate of the image signal output from the imaging device 522, thereby increasing the exposure time in the imaging device 522. Furthermore, the control section 94 may control the image generation section 93 (particularly image processor 931) to add data (pixel values) of pixels of two or more imaging devices in the process of generating a captured image.

On the other hand, in a case of increasing the output frame rate of the imaging device 522, the control section 94 may control the imaging device 522 and the signal processor 523 to perform pixel data thinning and area-specific reading. In this case, data (pixel values) of only some of the plurality of pixels included in the imaging device 522 is used for image generation.

The input section 95 functions as an instruction receiving portion that receives an instruction from the user, receives an instruction from the user under the control of the control section 94, and transmits the instruction to the control section 94. The input section 95 can take any form, and may be configured as a device (e.g., touch panel, operation button, or the like) directly operated by the user.

Alternatively, the input section 95 may be configured as a connection portion to which a device (e.g., mouse, keyboard, or portable device) operated by the user is connected in a wired or wireless manner. For example, an instruction from the user input via an operation portion of the light source device 10, an operation portion of the camera head 50, an operation portion of the insertion device 20, and an operation portion of the display device 70 may be transmitted to the control section 94 via the input section 95 of the control device 90.

The output section 96 outputs various types of information under the control of the control section 94. The output section 96 can take any form, and may be provided as, for example, a speaker, a printer, a communication device, and/or an application.

The storage section 97 writes and reads various data by the control section 94, and stores, for example, a program executed by the control section 94, information necessary for processing of the control section 94, and the like.

### [Medical observation method]

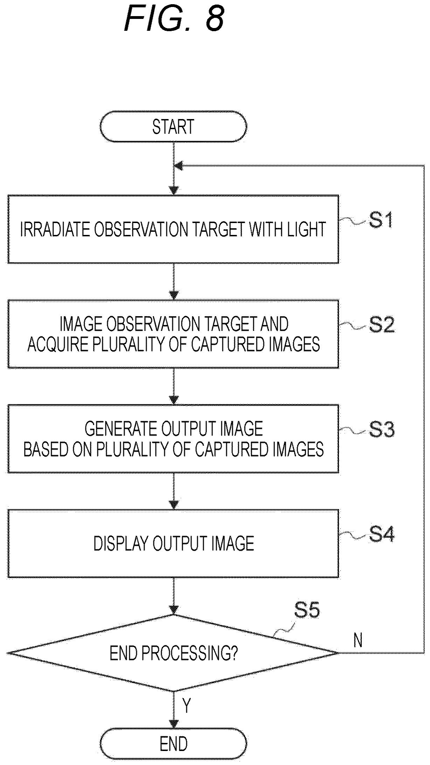

Fig. 8 is a flowchart illustrating an example of a medical observation method performed by the medical observation system 100.

First, under the control of the control section 94 of the control device 90, while the observation target is irradiated with light emitted from the light source device 10 and output from the insertion device 20 (S1 in Fig. 8), imaging of the observation target by the camera head 50 is performed, and a plurality of captured images is acquired (S2).

As described above, the light with which the observation target is irradiated may include visible light (e.g., broadband light such as white light) and excitation light (narrowband light in a visible light wavelength band or narrowband light in an invisible light wavelength band). The plurality of captured images may include a captured image based on visible light reflected by the observation target and a captured image based on fluorescence from the observation target.

The specific manner of light emission and imaging is not limited. For example, light emission of a plurality of types of light in the light source device 10 and light reception (exposure) of a plurality of types of light in the imaging device 522 of the camera head 50 may be performed intermittently, may be performed successively (continuously), may be performed in a time division manner, or may be performed simultaneously.

Then, under the control of the control section 94, the image generation section 93 generates an output image on the basis of the plurality of captured images (S3), and the output image is displayed on the display device 70 (S4). A user such as a doctor can confirm the state and characteristics of the tissue of the subject to be observed by viewing the output image displayed on the display device 70.

The above-described series of processing (S1 to S4) is repeatedly continued while the end of the processing is not instructed from the user (N in S5), and is ended when the end of the processing is instructed from the user (Y in S5). The control section 94 can receive such a processing end instruction from the user via the input section 95 (see Fig. 7).

Regarding the output image displayed on the display device 70 as described above, for example, for a captured image (i.e., "normal light-captured image") acquired by capturing an image of an observation target under irradiation of white light, high-resolution imaging may be required in order to provide a high-definition image. On the other hand, since the intensity of fluorescence from the observation target is not necessarily strong, high-sensitivity imaging may be required to acquire a captured image based on fluorescence. Although both high-resolution imaging and high-sensitivity imaging are satisfied by using a high-resolution and high-sensitivity imaging device, such a high-performance imaging device is expensive, and it may be difficult to prepare such a high-performance imaging device.

On the other hand, it is also possible to perform high-resolution imaging and high-sensitivity imaging by using a high-sensitivity imaging device and a high-resolution imaging device prepared as separate imaging devices. For example, observation light from a subject may be guided to a high-sensitivity imaging device or a high-resolution imaging device on the basis of the wavelength band. That is, desired high-resolution imaging and high-sensitivity imaging can be performed by guiding observation light in a certain wavelength band (e.g., light flux including reflected light of white light) to a high-resolution imaging device and guiding observation light in another wavelength band (e.g., light flux including fluorescence) to a high-sensitivity imaging device.

In a case where a plurality of imaging devices having different characteristics is used as described above, sensor sizes of the imaging devices may be different. In such a case, the range (angle of view) of the observation target captured in the captured image may be different among the plurality of imaging devices, and the number of pixels (size) of the captured image may be different among the imaging devices. In a case where such a captured image is displayed while being switched on one display device, the user needs to perform visual field matching work between displayed images in his/her head. With such visual field matching between the display images, observing the observation target while matching or associating the position and range of the observation target (e.g., identification target such as lesion) between the display images is troublesome work that puts a burden on the user, and accurate recognition of the identification target can be inhibited.

In addition, the plurality of captured images having different angles of view may include not only an observation target region included in common in the plurality of captured images but also an observation target region included in a certain captured image but not included in other captured images. For example, an observation target region included in a normal light-captured image may not be included in a fluorescence-captured image. In a superimposed image generated by superimposing and combining the normal light-captured image and the fluorescence-captured image, an observation target region included only in the normal light-captured image is represented as a normal light-captured image, but is not represented as a fluorescence-captured image. That is, the observation target region included only in the normal light-captured image can be recognized as a normal light-captured image by the user such as a doctor in the superimposed image. On the other hand, since the observation target region included only in the normal light-captured image is not included in a fluorescence-captured image, even if the region contains a specific substance and emits fluorescence, the fluorescence-captured image of the region is not included in the superimposed image. Therefore, even if the observation target region included only in the normal light-captured image emits fluorescence, the user who views the superimposed image may erroneously recognize the region as a "tissue that does not emit fluorescence (i.e., tissue that does not contain specific substance)" and erroneously diagnose the region.

As described above, in a case where a plurality of captured images having different angles of view are superimposed, not only an observation target region (common image region) commonly included in all the captured images but also an observation target region (non-common image region) included in a certain captured image but not included in other captured images can be included in the superimposed image. It is not necessarily easy for a user such as a doctor to accurately distinguish such common image regions and non-common image regions in the superimposed image.

Therefore, in the output image generated on the basis of the plurality of captured images, it is desirable to generate the output image so as to be advantageous for the user to accurately recognize the image of the observation target region that is included in common in the plurality of captured images.

### [Image generation method]

Next, a specific example of an image generation method regarding generation of an output image will be described.

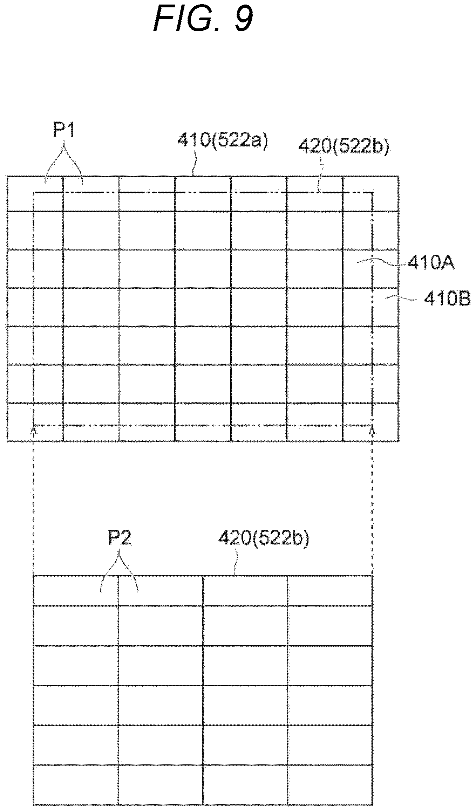

Fig. 9 illustrates an example of a physical size relationship between an imaging region 410 (effective pixel region) of the first imaging device 522a and an imaging region 420 (effective pixel region) of the second imaging device 522b. In Fig. 9, a rectangle superimposed on the imaging region 410 (solid line) of the first imaging device 522a and indicated by a two-dot chain line indicates a range corresponding to the imaging region 420 of the second imaging device 522b illustrated in Fig. 9.

A first image (captured image) is generated on the basis of an image signal (pixel data) output from the imaging region 410 of the first imaging device 522a, and a second image (captured image) is generated on the basis of an image signal output from the imaging region 420 of the second imaging device 522b.

In the example illustrated in Fig. 9, the first imaging device 522a and the second imaging device 522b are imaging devices of different types, and the imaging region 410 of the first imaging device 522a has a physically larger size than the imaging region 420 of the second imaging device 522b.

The angle of view of the first imaging device 522a is wider than the angle of view of the second imaging device 522b, and the range of the observation target imaged by the first imaging device 522a is wider than the range of the observation target imaged by the second imaging device 522b. That is, the entire range of the observation target imaged by the second imaging device 522b is included in the range of the observation target imaged by the first imaging device 522a. Therefore, the observation target included in the first image includes the entire range of the observation target included in the second image.

As described above, the first image is an image obtained by imaging the first range of the observation target using the first imaging device 522a. On the other hand, the second image is an image obtained by imaging a second range different from the first range of the observation target using the second imaging device 522b different in type from the first imaging device 522a. Here, one of the first range and the second range is wider than the other, and in the example illustrated in Fig. 9, the second range is included in the first range. In addition, the "type" mentioned here is, for example, a physical size, an image size, or a model number of the imaging device, but is not limited thereto. Then, an output image is generated on the basis of the first image and the second image by an image generation method performed by the control device 90 (image generation device) as described later. The output image generated in this manner is, for example, an image obtained by performing processing of replacing or deleting one or more pixel signals among the pixel signals of the first image and the second image on the basis of the first range and the second range of the observation target.

Light from a part of the observation target, which is included in both the first image and the second image, is incident on both the first imaging device 522a and the second imaging device 522b. On the other hand, light from a part of the observation target, which is captured in the first image but not in the second image, is incident on the first imaging device 522a, but is not incident on the second imaging device 522b.

As described above, the imaging region 410 of the first imaging device 522a includes a common light receiving region 410A that receives light from the common part of the observation target and a non-common light receiving region 410B that receives light from the non-common part of the observation target. On the other hand, the imaging region 420 of the second imaging device 522b includes only the common light receiving region 410A that receives light from the common part of the observation target.

As described above, the common light receiving region 410A of the first imaging device 522a is a region optically corresponding to the entire imaging region 420 of the second imaging device 522b, and the same part as the part of the observation target imaged by the second imaging device 522b is imaged in the common light receiving region 410A. On the other hand, the non-common light receiving region 410B of the first imaging device 522a is a region that does not optically correspond to the imaging region 420 of the second imaging device 522b, and a part of the observation target that is not imaged by the second imaging device 522b is imaged in the non-common light receiving region 410B.

As described above, the first imaging device 522a and the second imaging device 522b respectively acquire the first image and the second image in which the same observation target is captured and in which the ranges of the captured observation target are different from each other.

In the example illustrated in Fig. 9, the number of pixels P1 of the first imaging device 522a (imaging region 410) is larger than the number of pixels P2 of the second imaging device 522b (imaging region 420). As an example, the number of pixels of the first imaging device 522a may be 3840×2160 corresponding to 4K resolution, and the number of pixels of the second imaging device 522b may be 1920×1080 corresponding to full high definition (HD).

Note, however, that the number of pixels of the first imaging device 522a and the number of pixels of the second imaging device 522b are not limited. The number of pixels of the imaging device is related not only to the physical size of the imaging region but also to the size of each pixel (i.e., pixel size). Therefore, the number of pixels of the first imaging device 522a may be the same as the number of pixels of the second imaging device 522b, or may be smaller than the number of pixels of the second imaging device 522b. As described above, the relative number of pixels between the first imaging device 522a and the second imaging device 522b is not limited.

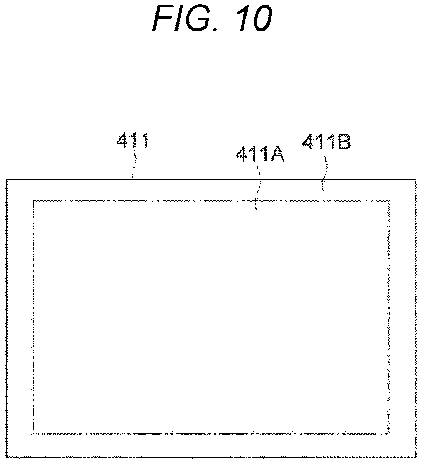

Fig. 10 is a schematic diagram illustrating an example of a first image 411 generated on the basis of an image signal from the first imaging device 522a illustrated in Fig. 9.

The first image 411 illustrated in Fig. 10 includes a common image region 411A and a non-common image region 411B adjacent to the common image region 411A. An image based on an image signal output from the common light receiving region 410A (see Fig. 9) of the first imaging device 522a is captured in the common image region 411A, and an image based on an image signal output from the non-common light receiving region 410B (see Fig. 9) is captured in the non-common image region 411B.

Therefore, the common image region 411A includes an image of a part of the observation target, which is captured by both the first imaging device 522a and the second imaging device 522b and is included in the entire second image captured and acquired by the second imaging device 522b. On the other hand, the non-common image region 411B includes an image of a part of the observation target, which is not included in the second image.

Note that in the example illustrated in Fig. 10, the non-common image region 411B surrounds the entire common image region 411A (up, down, left, and right), but the range of the non-common image region 411B is not limited, and the non-common image region 411B does not necessarily surround the entire common image region 411A. It is not always necessary to specify specific ranges of the common image region 411A and the non-common image region 411B in the first image 411. Therefore, the image generation section 93 (e.g., superimposed image generator 934) does not always need to perform the processing of specifying the ranges of the common image region 411A and the non-common image region 411B in the first image 411.

In each embodiment described below, a case where the two imaging devices 522a and 522b illustrated in Fig. 9 are provided in the imaging section 52 of the camera head 50 will be described. That is, in each of the following embodiments, an output image is generated from the captured image (i.e., first image and second image) generated on the basis of the image signal output from each of the two imaging devices 522a and 522b, and the output image is displayed on the display device 70.

Furthermore, in the following embodiments, the first imaging device 522a images an observation target irradiated with visible light (particularly white light; first light) in the first wavelength band from the first light source 11 to acquire a captured image (normal light-captured image; first image). On the other hand, the second imaging device 522b images an observation target irradiated with excitation light that is excitation light (second light) from the second light source 12 and that excites a specific substance so as to emit visible fluorescence or fluorescence that is invisible light to acquire a captured image (fluorescence-captured image; second image). The excitation light is light in a wavelength band at least partially different from the first wavelength band. Therefore, the first image acquired by the first imaging device 522a includes a captured image based on reflected light of visible light (white light) from the observation target. On the other hand, the second image acquired by the second imaging device 522b includes a captured image based on fluorescence (i.e., visible fluorescence having at least partial wavelength different from first wavelength band, or fluorescence in invisible light wavelength band) from the observation target irradiated with the excitation light.

Note that the present disclosure technology is not limited to the following embodiments, and can be appropriately applied to other embodiments not shown below. For example, the imaging section 52 does not necessarily need to include the two imaging devices 522a and 522b illustrated in Fig. 9, and, for example, even in a case where the imaging section 52 includes three or more imaging devices 522, the technologies illustrated in the following embodiments can be effectively applied. Furthermore, the wavelength characteristic of the light received by the imaging device 522 is not limited. For example, even in a case where two or more types of fluorescence emitted from an observation target irradiated with excitation light and having different wavelength bands are received by one or a plurality of imaging devices 522, the technology described in each of the following embodiments can be effectively applied.

### [First embodiment]

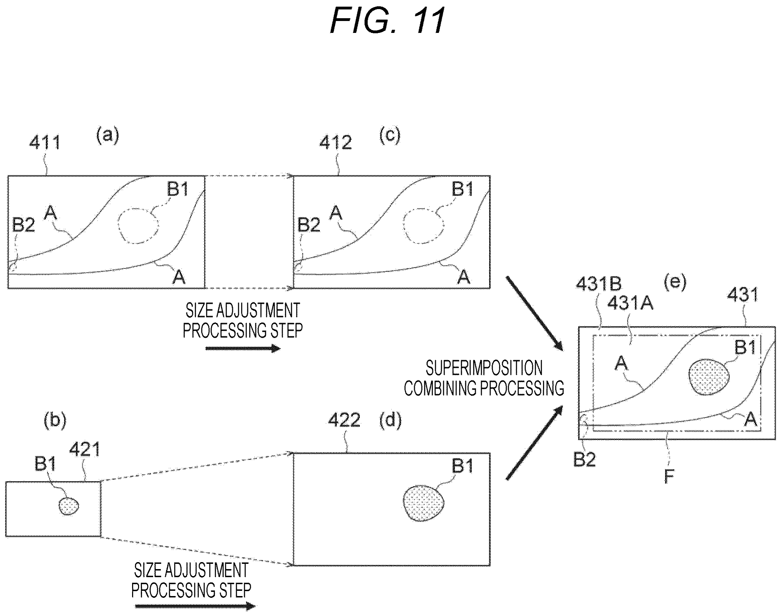

Fig. 11 is a diagram illustrating an example of generation processing of first images 411 and 412, second images 421 and 422, and a superimposed image 431 in a first embodiment.

Parts (a) and (c) of Fig. 11 illustrate the first images 411 and 412 generated on the basis of the image signal from a first imaging device 522a, where part (a) illustrates the first image 411 before a size adjustment processing step, and part (c) illustrates the first image 412 after the size adjustment processing step. Parts (b) and (d) of Fig. 11 illustrate the second images 421 and 422 generated on the basis of the image signal from a second imaging device 522b, where part (b) illustrates the second image 421 before the size adjustment processing step, and part (d) illustrates the second image 422 after the size adjustment processing step. Part (e) of Fig. 11 illustrates a superimposed image 431 generated on the basis of the first image 412 and the second image 422 after the size adjustment processing step. The processing of generating the various images illustrated in Fig. 11 is appropriately performed by an image generation section 93 under the control of a control section 94 in a control device 90 (see Fig. 7).

As an example, an organ having fluorescent sites B1 and B2 as identification targets is imaged by the first imaging device 522a and the second imaging device 522b as an observation target. Note that parts (a) and (c) of Fig. 11 illustrate the fluorescent sites B1 and B2 surrounded by a two-dot chain line, but the drawing is merely a reference diagram illustrating the corresponding ranges and positions of the fluorescent sites B1 and B2. That is, the first images 411 and 412 in parts (a) and (c) of Fig. 11 do not include the fluorescent sites B1 and B2 as identifiable images. Similarly, part (e) of Fig. 11 illustrates a diagram in which the fluorescent site B2 is surrounded by a two-dot chain line, but the diagram is merely a reference diagram illustrating the corresponding range and position of the fluorescent site B2. That is, the superimposed image 431 in part (e) of Fig. 11 does not include the fluorescent site B2 as an identifiable image. Note, however, that as will be described later, the superimposed image 431 in part (e) of Fig. 11 includes the fluorescent site B1 as an identifiable image.

In the present embodiment, in the size adjustment processing step, the captured images 412 and 422 (see parts (c) and (d) of Fig. 11) having desired sizes are generated from the captured images 411 and 421 (see parts (a) and (b) of Fig. 11) based on the image signal output from an imaging device 522. Then, an output image is generated on the basis of the first image 412 and the second image 422 after the size adjustment processing step.

Specifically, enlargement/reduction processing is performed on the first image 411 and the second image 421, and the sizes (the number of pixels) of both or one of the first image 411 and the second image 421 are adjusted, so that the first image 412 and the second image 422 after the size adjustment processing step are provided. In enlargement processing, pixel interpolation is performed, the number of pixels constituting the image is increased, and the overall size of the image is increased. In reduction processing, pixel thinning is performed, the number of pixels constituting the image is reduced, and the overall size of the image is reduced.

The enlargement/reduction magnification in the enlargement/reduction processing can be determined in consideration of generation of the superimposed image 431 to be described later. Specifically, the enlargement/reduction magnification is determined such that the size of the region (common image region) of the observation target (including identification target) commonly captured in the first image 411 and the second image 421 is consistent between the first image 411 and the second image 421. As a result, size adjustment of one or both of the first image 411 and the second image 421 is performed such that the number of pixels of the common image region is consistent between the first image 412 and the second image 422.

In the examples of parts (a) and (b) of Fig. 11, before the size adjustment processing step, the size (number of pixels) of the second image 421 based on the image signal output from the second imaging device 522b is smaller than the size of the first image 411 based on the image signal output from the first imaging device 522a. Therefore, the enlargement magnification of the enlargement processing on the second image 421 is determined such that the entire second image 422 has the same size (number of pixels) as the common image region (see reference numeral "411A" in Fig. 10) of the first image 412 after the size adjustment processing step.

Accordingly, after the size adjustment processing step, the common image region of the first image 412 has the same angle of view as the entire second image 422, and the observation target is shown in the common image region of the first image 412 and the second image 422 in the same range and the same size.

Note that in the example illustrated in Fig. 11, the first image 411 may be enlarged or reduced, or may be not enlarged nor reduced in the size adjustment processing step. Therefore, the first images 411 and 412 (see parts (a) and (c) of Fig. 11) based on the image signal from the first imaging device 522a may have the same number of pixels before and after the size adjustment processing step. In this case, the enlargement/reduction processing on the first image 411 is unnecessary, and the size adjustment processing of the first image 411 is substantially omitted in the size adjustment processing step. Note that in this case, too, the first image before the size adjustment processing step is denoted by reference numeral "411", and the first image after the size adjustment processing step is denoted by reference numeral "412".

As described above, the range of the observation target appearing in the second images 421 and 422 is narrower than the range of the observation target appearing in the first images 411 and 412, and an observation target region (non-common image region 411B in Fig. 10) not appearing in the second images 421 and 422 is included in the first images 411 and 412. Therefore, the total number of pixels is not consistent between the first image 412 and the second image 422 after the size adjustment processing step, and the number of pixels of the second image 422 is smaller than the number of pixels of the first image 412.

Then, the superimposed image 431 is generated on the basis of the first image 412 and the second image 422 after the size adjustment processing step (see part (e) of Fig. 11).

That is, the first image 412 and the second image 422 are superimposed and combined such that the size and position of the observation target region commonly included in the first image 412 and the second image 422 coincide with each other. As an example, the superimposition combining processing of the first image 412 and the second image 422 may be performed such that the center of the second image 422 is aligned with the center of the common image region of the first image 412.

As described above, after the size adjustment processing step, the number of pixels of the common image region of the first image 412 coincides with the number of pixels of the entire second image 422. As described above, since the observation target regions commonly captured between the first image 412 (particularly common image region) and the second image 422 correspond to each other in units of pixels, the superimposition combining processing of the first image 412 and the second image 422 can be appropriately performed in units of pixels.

As described above, the superimposed image 431 is generated on the basis of the first image 412 and the second image 422 adjusted so that the image sizes (number of pixels) of the observation target region (particularly fluorescent site B1 as identification target) coincide with each other, whereby the identification target is appropriately displayed in the superimposed image 431.

Note that as described above, even after the size adjustment processing step, the overall size of the first image 412 is larger than the overall size of the second image 422 (see parts (c) and (d) of Fig. 11). Therefore, the superimposed image 431 includes a superimposing region 431A to which an image based on the first image 412 and the second image 422 is allocated, and a non-superimposing region 431B to which an image based on the first image 412 but not based on the second image 422 is allocated.

The superimposed combined image of the observation target region, which is commonly included in the first image 412 and the second image 422, is allocated to the superimposing region 431A of the superimposed image 431. On the other hand, an image of the observation target region (i.e., non-common image region of first image 412 (see reference numeral "411B" in Fig. 10)), which is included only in the first image 412 and is not included in the second image 422, is allocated to the non-superimposing region 431B adjacent to the superimposing region 431A. Note that in the example illustrated in part (e) of Fig. 11, the non-superimposing region 431B surrounds the entire superimposing region 431A, but the range of the non-superimposing region 431B is not limited, and the non-superimposing region 431B does not necessarily surround entire superimposing region 431A.

As described above, the second image 422 is reflected in the image allocated to the superimposing region 431A of the superimposed image 431, but is not reflected in the image allocated to the non-superimposing region 431B.

In the example illustrated in Fig. 11, the reflected light of the white light with which the fluorescent sites B1 and B2 are irradiated has wavelength characteristics similar to those of the reflected light of the white light with which the sites around the fluorescent sites B1 and B2 are irradiated. Therefore, in the first images 411 and 412 which are normal light-captured images, an organ A as the observation target is included as an identifiable image, but the fluorescent sites B1 and B2 are not included as an identifiable image. Therefore, it is difficult for the user to visually distinguish the fluorescent sites B1 and B2 from the surrounding sites in the first images 411 and 412 (normal light-captured images) acquired by the first imaging device 522a (see part (a) of Fig. 11).

On the other hand, the fluorescent sites B1 and B2 of this example emit fluorescence that can be imaged by the second imaging device 522b by being irradiated with excitation light (second light) from the second light source 12. Therefore, the organ A as the observation target is not included as an identifiable image in the second image 421 (see part (b) of Fig. 11) which is a fluorescence-captured image. On the other hand, the second image 421 includes the fluorescent site (particularly fluorescent site B1 located in observation target region, which is commonly included in first image 412 and second image 422) as an identifiable image. Therefore, the user can visually identify the fluorescent site B1 from the surrounding site in the second image 421 (fluorescence-captured image) acquired by the second imaging device 522b.

On the other hand, the fluorescent site B2 existing in the region of the organ, which is shown as the normal light-captured image in the non-superimposing region 431B, is not shown as a fluorescence-captured image in the superimposed image 431. Therefore, the user viewing the superimposed image 431 may erroneously recognize that such a fluorescent site does not exist even if the fluorescent site exists in the region of the organ shown in the non-superimposing region 431B.

Furthermore, a boundary between the superimposing region 431A and the non-superimposing region 431B in the superimposed image 431 is not necessarily visually clear. Note that in part (e) of Fig. 11, a boundary F between the superimposing region 431A and the non-superimposing region 431B is indicated by a two-dot chain line, but the boundary F is a virtual line added for convenience and is not a line explicitly displayed from the beginning in the superimposed image 431. Therefore, it is not always easy for the user to clearly identify the image range related to the second image 422 in the superimposed image 431, and there is a possibility that the user erroneously recognizes at least a partial range of the non-superimposing region 431B as the superimposing region 431A.

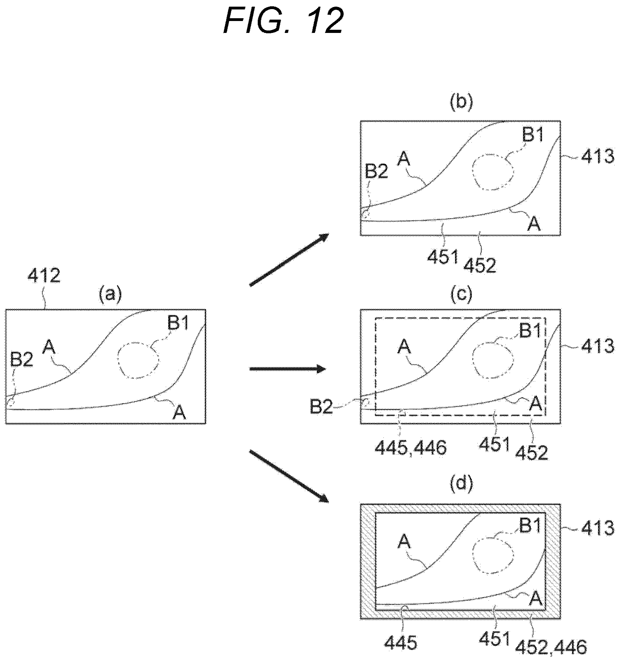

Figs. 12 to 14 are diagrams illustrating an example of output image generation processing according to the first embodiment.

Fig. 12 illustrates an example of a first output image 413 generated mainly from the first image 412 (normal light-captured image) which is a captured image by the first imaging device 522a. Part (a) of Fig. 12 illustrates the first image 412 that is the original image of the first output image 413 (see parts (b) to (d) of Fig. 12). The processing of generating various images illustrated in Fig. 12 is appropriately performed by the image generation section 93 under the control of the control section 94 in the control device 90 (see Fig. 7).

In the example illustrated in Fig. 12, the first output image 413 based on the first image 412 is generated, and can be displayed on the display device 70 simultaneously or switchably with other images.

The first output image 413 of the present embodiment includes a first image region 451 and a second image region 452 different from the first image region 451. In particular, the first image region 451 is generated on the basis of the first image 412 and the second image 422, and an image of the observation target region, which is commonly included in the first image 412 and the second image 422, is allocated. Here, the first image region 451 is "generated on the basis of the first image 412 and the second image 422" can mean that the range and size of the image of the observation target region allocated to the first image region 451 are determined on the basis of the first image 412 and the second image 422.

For example, first output image 413 based on the first image 412 may be the same image as the first image 412 (see part (a) of Fig. 12) of the original image (see part (b) of Fig. 12). In this case, the boundary between the first image region 451 and the second image region 452 is not indicated in the first output image 413, and the user can visually recognize the first output image 413 through the display device 70 without being conscious of the boundary.

Alternatively, as illustrated in parts (c) and (d) of Fig. 12, pixel replacement processing may be performed such that a boundary emphasis image 446 indicating a boundary 445 of the first image region 451 is included in the first output image 413.

The pixel replacement processing described herein includes processing in general of changing the value of one or a plurality of pixels. The pixel values before and after the pixel replacement processing may be values unrelated to each other, or the pixel value after the pixel replacement processing may be determined on the basis of the pixel value before the pixel replacement processing. Therefore, pixel combination processing in which pixel values after pixel replacement processing are derived on the basis of a plurality of pixel values before the pixel replacement processing is also included in the pixel replacement processing.

In the first output image 413 of the example illustrated in part (c) of Fig. 12, as indicating the boundary 445 between the first image region 451 and the second image region 452, the boundary emphasis image 446 having a line shape (e.g., dotted line shape) extends along the boundary 445. For example, the boundary emphasis image 446 can be included in the first output image 413 by performing pixel replacement processing on a corresponding pixel of the first output image 413 such that the corresponding pixel exhibits a specific color indicating the boundary emphasis image 446.

The boundary emphasis image 446 extending along the boundary 445 of the first image region 451 as described above may be located only in the first image region 451, only in the second image region 452, or both in the first image region 451 and the second image region 452. Note that it is preferable to reduce the occupied area (e.g., line width) of the boundary emphasis image 446 from the viewpoint of suppressing reduction in the amount of information in the first output image 413.

On the other hand, in the example illustrated in part (d) of Fig. 12, a unique image is allocated to the second image region 452 as the boundary emphasis image 446, whereby the boundary 445 of the first image region 451 is indicated in the first output image 413.

The boundary emphasis image 446 allocated to the second image region 452 is an image that can be visually distinguished and identified from the image (i.e., first image 412) allocated to the first image region 451, and can have an arbitrary color and pattern. Therefore, the boundary emphasis image 446 may be, for example, a mask image of a single color, an image of a diagonal line pattern, or an image of a checkered pattern. In a case where the switching display of the first output image 413 and another image (e.g., superimposed image) is performed, since the size of the observation target does not change between the display images, the user such as a doctor can smoothly advance medical practice such as surgery.

Moreover, in the second image region 452, an image based on the first image 412 (e.g., image of non-common image region) may be shown together with the boundary emphasis image 446. For example, an image (translucent image) of a non-common image region of the first image 412 combined with the boundary emphasis image 446 by alpha blending may be allocated to the second image region 452 of the first output image 413.

As described above, in the first output image 413 generated on the basis of the normal light-captured image, the organ A is included as an identifiable image, but the fluorescent sites B1 and B2 are not included as an identifiable image. Therefore, from the first output image 413, the user can identify the organ A but finds it difficult to identify the fluorescent sites B1 and B2.

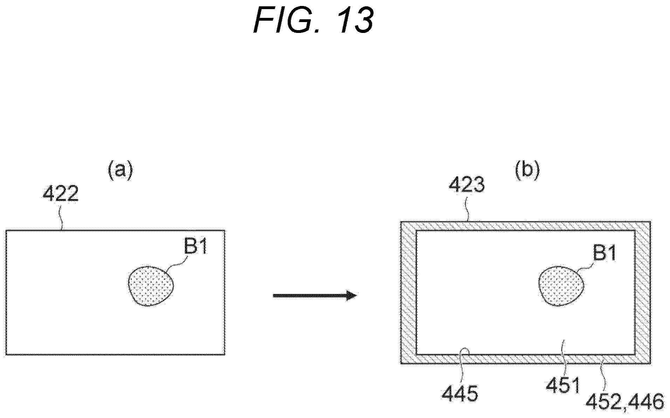

Fig. 13 illustrates an example of a second output image 423 generated mainly from the second image 422 (fluorescence-captured image) which is a captured image by the second imaging device 522b. Part (a) of Fig. 13 illustrates the second image 422 that is the original image of the second output image 423 (see part (b) of Fig. 13). The processing of generating various images illustrated in Fig. 13 is appropriately performed by the image generation section 93 under the control of the control section 94 in the control device 90 (see Fig. 7).

In the example illustrated in Fig. 13, the second output image 423 based on the second image 422 is generated, and can be displayed on the display device 70 simultaneously or switchably with other images.

As illustrated in part (b) of Fig. 13, the second output image 423 based on the second image 422 may have the first image region 451 to which the second image 422 is allocated, and the second image region 452 to which a specific image that acts as the boundary emphasis image 446 is allocated.

In this case, the boundary emphasis image 446 allocated to the second image region 452 is an image that can be visually distinguished and identified from the image (i.e., second image 422) allocated to the first image region 451, and can have an arbitrary color and pattern. For example, an image (e.g., black mask image) unrelated to the first image 412 and the second image 422 may be used as the boundary emphasis image 446. In a case where the switching display of the second output image 423 and another image (e.g., superimposed image) is performed, since the size of the observation target does not change between the display images, the user such as a doctor can smoothly advance medical practice such as surgery.

The image processing method for generating such a second output image 423 is not limited. For example, the second output image 423 may be generated by performing pixel addition processing of adding the boundary emphasis image 446 around the second image 422 by the image generation section 93.

In the second output image 423 generated on the basis of the second image 422 which is the fluorescence captured image, the organ A as the observation target is not included as an identifiable image, but the fluorescent site (fluorescent site B1 located in first image region 451) is included as an identifiable image. Therefore, from the second output image 423, the user finds it difficult to identify the organ A, but can identify the fluorescent site B1.

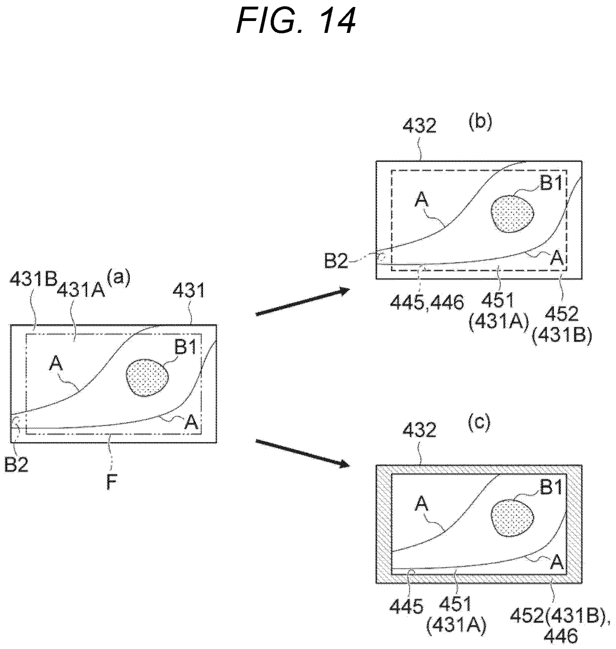

Fig. 14 illustrates an example of a superimposed output image 432 generated from the first image 412 (normal light-captured image) and the second image 422 (fluorescence-captured image) which are captured images by the first imaging device 522a and the second imaging device 522b. Part (a) of Fig. 14 illustrates the superimposed image 431 that is an original image of the superimposed output image 432 (see parts (b) and (d) of Fig. 14). The processing of generating various images illustrated in Fig. 14 is appropriately performed by the image generation section 93 under the control of the control section 94 in the control device 90 (see Fig. 7).

In the example illustrated in Fig. 14, the superimposed output image 432 based on the superimposed image 431 is generated, and can be displayed on the display device 70 simultaneously or switchably with other images.

As illustrated in parts (b) and (c) of Fig. 14, the superimposed output image 432 based on the superimposed image 431 may have the first image region 451 to which the superimposed image is allocated and the second image region 452 to which an image different from the first image region 451 is allocated. In particular, the pixel replacement processing may be performed such that the boundary emphasis image 446 indicating the boundary 445 of the first image region 451 is included in the superimposed output image 432.

As described above, the superimposed image allocated to the first image region 451 of the superimposed output image 432 is generated on the basis of the image of the common image region in the first image 412 and the image of the common image region in the second image 422 having the same number of pixels.

In the example illustrated in part (b) of Fig. 14, the boundary emphasis image 446 having a line shape (e.g., dotted line shape) extends along the boundary 445 between the first image region 451 and the second image region 452. For example, the boundary emphasis image 446 can be included in the superimposed output image 432 by performing pixel replacement processing on a corresponding pixel of the superimposed output image 432 so that the corresponding pixel exhibits a specific color indicating the boundary emphasis image 446. The boundary emphasis image 446 extending along the boundary 445 of the first image region 451 as described above may be located only in the first image region 451, only in the second image region 452, or both in the first image region 451 and the second image region 452.

On the other hand, in the example illustrated in part (c) of Fig. 14, a unique image is allocated to the second image region 452 as the boundary emphasis image 446, whereby the boundary 445 of the first image region 451 is indicated in the superimposed output image 432. The boundary emphasis image 446 allocated to the second image region 452 is an image that can be visually distinguished and identified from the image allocated to the first image region 451 (i.e., superimposed image of first image 412 and second image 422), and may have any color and pattern. In particular, using an image (e.g., black mask image) unrelated to the first image 412 and the second image 422 as the boundary emphasis image 446 is advantageous in preventing erroneous recognition of the superimposed output image 432.

In second image region 452, an image based on the first image 412 (e.g., image of non-common image region 411B (see Fig. 10)) may be shown together with boundary emphasis image 446.

The image processing method for generating such a superimposed output image 432 is not limited. For example, the superimposed output image 432 may be generated by performing pixel replacement processing of replacing data of a plurality of pixels of the superimposed image 431 (particularly plurality of pixels constituting non-superimposing region 431B) with data of the boundary emphasis image 446. Alternatively, the superimposed output image 432 may be generated by performing pixel replacement processing of combining the superimposed image 431 (particularly, image of non-superimposing region 431B) and the boundary emphasis image 446.

For example, in a case where the above-described switching display of the second output image 423 (see Fig. 13) and the superimposed output image 432 (see Fig. 14) is performed in the display device 70, the image generation section 93 of the control device 90 outputs the second output image 423 and the superimposed output image 432 to the display device 70. Here, the superimposed output image 432 is "an image obtained by performing processing of replacing or deleting one or more pixel signals among the pixel signals of the first image 412 and the second image 422 on the basis of a first range that is a range of an observation target imaged by the first imaging device 522a for acquiring the first images 411 and 412 and a second range that is a range of an observation target imaged by the second imaging device 522b for acquiring the second images 421 and 422". Additionally, the second output image 423 is an image generated by adding one or more pixel signals (i.e., pixel data (pixel values)) to an image generated from at least some of the pixel signals of the second image 422.

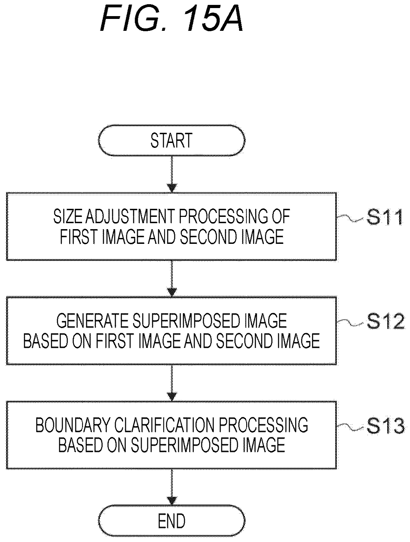

Figs. 15A to 15C are flowcharts illustrating an example of generation processing of the superimposed output image 432.

Fig. 15A is a flowchart of an example of generation processing corresponding to the above-described examples illustrated in Figs. 11 to 14. That is, after the execution of the size adjustment processing step for the first image 411 and the second image 421 (S11 in Fig. 15A; parts (a) to (d) of Fig. 11), the superimposed image 431 is generated on the basis of the first image 412 and the second image 422 after the size adjustment processing (S12; part (e) of Fig. 11). Then, the boundary emphasis image 446 indicating the boundary of the superimposing region 431A (i.e., boundary 445 of first image region 451 of superimposed output image 432) is applied to the superimposed image 431, thereby generating the superimposed output image 432 (S13; Fig. 14).

Note, however, that the superimposed output image 432 can also be generated by another processing flow.

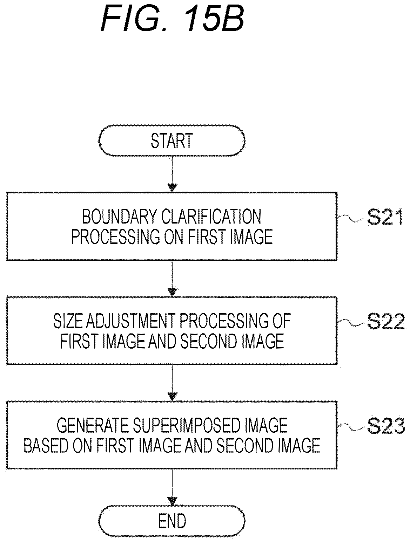

For example, as illustrated in Fig. 15B, after boundary clarification processing for clarifying the boundary of the common image region 411A (see Fig. 10) is performed on the first image 411 (S21), the size adjustment processing step may be performed on the first image 411 and the second image 421 (S22). In this case, the superimposed image 431 (S23) generated on the basis of the first image 412 and the second image 422 after the size adjustment processing step can be used as the superimposed output image 432 (see Fig. 14).

In this example, the "image that emphasizes the boundary between the common image region 411A and the non-common image region 411B" applied to the first image 411 by the boundary clarification processing (S21) on the first image 411 finally configures the boundary emphasis image 446 in the superimposed output image 432. The "image that emphasizes the boundary between the common image region 411A and the non-common image region 411B" mentioned here is not limited, and may be a line-shaped image extending along the boundary between the common image region 411A and the non-common image region 411B. Alternatively, a specific image that can be visually distinguished and identified from the image allocated to the common image region 411A may be allocated to the non-common image region 411B as the "image that emphasizes the boundary between the common image region 411A and the non-common image region 411B".

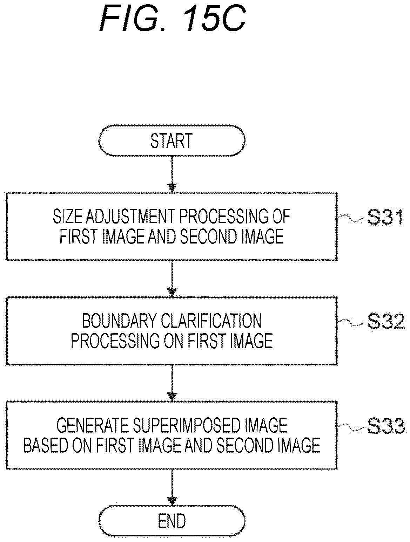

Alternatively, as illustrated in Fig. 15C, after the size adjustment processing step is performed on the first image 411 and the second image 421 (S31), the boundary clarification processing (S32) for clarifying the boundary of the common image region 411A may be performed on the first image 412. In this case, the superimposed image 431 generated on the basis of the first image 412 and the second image 422 after the boundary clarification processing (S33) can be used as the superimposed output image 432 (see Fig. 14).

Also in this example, the "image that emphasizes the boundary between the common image region 411A and the non-common image region 411B" applied to the first image 412 by the boundary clarification processing (S32) performed on the first image 412 finally configures the boundary emphasis image 446 in the superimposed output image 432.

Note that in the size adjustment processing (e.g., enlargement processing) of the first image 411 and the second image 421, data of new pixels (interpolation pixels) may be generated on the basis of data of a plurality of pixels in the original image depending on the processing algorithm. In such size adjustment processing, if the pixel data of the above-described "image emphasizing the boundary" that is not originally included in the first image 411 and the second image 421 is used to create data of interpolation pixels, the boundary emphasis image 446 in the superimposed output image 432 may become unclear. Therefore, from the viewpoint of creating the clear boundary emphasis image 446 in the superimposed output image 432, it is preferable to perform the boundary clarification processing after the size adjustment processing (e.g., enlargement processing) of the first image 411 and the second image 421 (see Figs. 15A and 15C described above).

As described above, according to the present embodiment, in the first image region 451 of the output images 413, 423, and 432, an image based on both or one of the first images 411 and 412 and the second images 421 and 422, which are images of the observation target region and are commonly captured in the first images 411 and 412 and the second images 421 and 422, is allocated. Then, in the output images 413, 423, and 432, a boundary emphasis image 446 indicating the boundary 445 of the first image region 451 can be included.

The user can clearly grasp the boundary 445 of the first image region 451 in the output images 413, 423, and 432 on the basis of the boundary emphasis image 446 in the output images 413, 423, and 432. Therefore, in the output images 413, 423, and 432, the user can accurately recognize the image of the observation target region commonly included in the first images 411 and 412 and the second images 421 and 422.

In addition, the first output image 413, the second output image 423, and the superimposed output image 432 can be generated so as to have the same number of pixels and resolution as a whole. In particular, the number of pixels and resolution of the first image region 451, to which the image of the observation target region common to the first images 411 and 412 and the second images 421 and 422 is allocated, are made consistent between the first output image 413, the second output image 423, and the superimposed output image 432.

In this case, in the first image region 451 of each output image, the same range of the observation target is indicated by the same size. Therefore, even in a case where the plurality of output images 413, 423, and 432 is switched and displayed on the common display device 70, the user can identify the observation target in each output image naturally, and can easily visually compare the observation target between the output images.

Note that the image generation section 93 (display control portion 935 (see Fig. 7)) of the control device 90 may change the display angle of view of the output images 413, 423, and 432 in the display device 70 on the basis of an instruction of digital zoom from the user via the input section 95. In particular, in a mode in which the boundary emphasis image 446 is displayed in the second image region 452 and the second image region 452 is used as the mask region (see part (d) of Fig. 12, part (b) of Fig. 13, and part (c) of Fig. 14), in a case where the display angle of view of the output images 413, 423, and 432 is changed, the output images 413, 423, and 432 may be generated such that only the image of the first image region 451 inside the mask region is displayed at the angle of view based on the user's instruction without changing the display size of the mask region (second image region 452) in the display device 70.

Alternatively, in the display device 70, the entire display sizes of the output images 413, 423, and 432 including the mask region (second image region 452) may be changed, and the output images 413, 423, and 432 may be generated such that the output images 413, 423, and 432 are displayed at the angle of view based on the user's instruction. In this case, it is also possible to adjust the display angle of view of the output images 413, 423, and 432 so that the first image region 451 is displayed on the entire display device 70, leading to improvement of the visibility of the output images 413, 423, and 432.

### [Second embodiment]

In the present embodiment, the same or corresponding elements as those in the above-described first embodiment are denoted by the same reference numerals, and a detailed description thereof will be omitted.

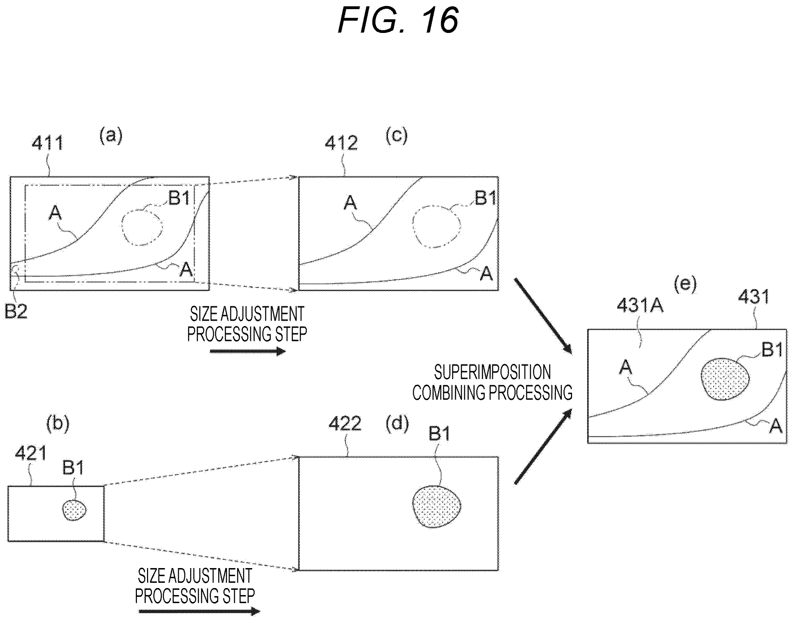

Fig. 16 is a diagram illustrating an example of generation processing of first images 411 and 412, second images 421 and 422, and a superimposed image 431 in a second embodiment.

Parts (a) and (c) of Fig. 16 illustrate the first images 411 and 412 generated on the basis of the image signal from a first imaging device 522a, where part (a) illustrates the first image 411 before a size adjustment processing step, and part (c) illustrates the first image 412 after the size adjustment processing step. Parts (b) and (d) of Fig. 16 illustrate the second images 421 and 422 generated on the basis of the image signal from a second imaging device 522b, where part (b) illustrates the second image 421 before the size adjustment processing step, and part (d) illustrates the second image 422 after the size adjustment processing step. Part (e) of Fig. 16 illustrates a superimposed image 431 generated on the basis of the first image 412 and the second image 422 after the size adjustment processing step. The processing of generating various images illustrated in Fig. 16 is appropriately performed by an image generation section 93 under the control of a control section 94 in a control device 90 (see Fig. 7).

As in the first embodiment described above, also in the present embodiment, as an example, an organ having fluorescent sites B1 and B2 as identification targets is imaged by the first imaging device 522a and the second imaging device 522b as an observation target. Note that parts (a) and (c) of Fig. 16 illustrate the fluorescent sites B1 and B2 surrounded by a two-dot chain line, but the drawing is merely a reference diagram illustrating the corresponding ranges and positions of the fluorescent sites B1 and B2. That is, the first images 411 and 412 in parts (a) and (c) of Fig. 16 do not include the fluorescent sites B1 and B2 as identifiable images.

In the present embodiment, too, in the size adjustment processing step, the captured images 412 and 422 (see parts (c) and (d) of Fig. 16) having desired sizes are generated from the captured images 411 and 412 (see parts (a) and (b) of Fig. 16) based on the image signal output from an imaging device 522. Then, an output image is generated on the basis of the first image 412 and the second image 422 after the size adjustment processing step.

In particular, in the present embodiment, in the first image 411, the image of the observation target region (common target region) (i.e., image of common image region 411A (see Fig. 10)) included in common in the first image 411 and the second image 421 is extracted, and the size adjustment processing of the extracted portion is performed. Here, the processing of extracting the image of the common image region 411A corresponds to pixel deletion processing of deleting the pixels of the non-common image region 411B from the first image 411.

Thereafter, enlargement/reduction processing is performed on both or one of the first image 411 (image of common image region 411A) and the second image 421 of the extracted portion, and the size (number of pixels) of both or one of the first image 411 and the second image 421 is adjusted (size adjustment processing step). Also in the present embodiment, the enlargement/reduction magnification in the enlargement/reduction processing is determined to be a magnification at which the size of the common image region is consistent between the first image 412 and the second image 422. Therefore, size adjustment of one or both of the first image 411 and the second image 421 is performed such that the number of pixels of the common image region is consistent between the first image 412 and the second image 422.

Then, an output image is generated on the basis of the "extracted first image 412 after the size adjustment processing step" and the "second image 422".

In the example illustrated in Fig. 16, before the size adjustment processing step, the overall size (number of pixels) of the second image 421 is smaller than the size of the partial image based on the image signal output from the common image region 411A of the first imaging device 522a in the first image 411.

Then, in the size adjustment processing step, the extracted partial image of the first image 411 (image of common image region 411A) is enlarged to constitute the entire first image 412. On the other hand, the entire second image 421 is enlarged. The first image 412 and the second image 422 after the size adjustment processing include only the image of the observation target region (common image region) that is commonly captured, and have the same number of pixels and resolution.

Then, the superimposed image 431 is generated on the basis of the first image 412 and the second image 422 after the size adjustment processing step (see part (e) of Fig. 16). That is, the first image 412 and the second image 422 are superimposed and combined such that the size and position of the observation target region commonly included in the first image 412 and the second image 422 coincide with each other.

As described above, the first image 412 and the second image 422 after the size adjustment processing step have the same size (number of pixels) for the same range of the observation target and have the same overall size and resolution. Therefore, the superimposed image 431 includes only a superimposing region 431A to which the images based on the first image 412 and the second image 422 are allocated, and does not include a non-superimposing region 431B (see part (e) of Fig. 11).

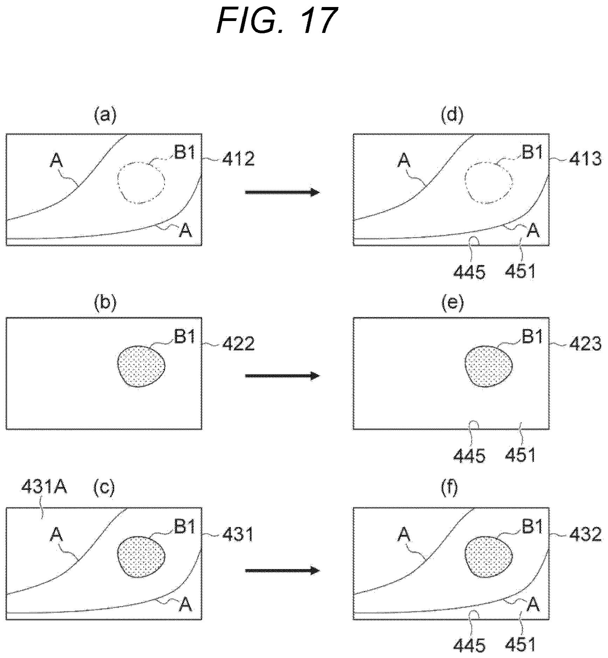

Fig. 17 is a diagram illustrating an example of output image generation processing according to the second embodiment.

Part (a) of Fig. 17 illustrates the first image 412 that is the original image of the first output image 413 (see part (d) of Fig. 17). Part (b) of Fig. 17 illustrates the second image 422 that is the original image of the second output image 423 (see part (e) of Fig. 17). Part (c) of Fig. 17 illustrates the superimposed image 431 that is the original image of the superimposed output image 432 (see part (f) of Fig. 17). The processing of generating various images illustrated in Fig. 17 is appropriately performed by the image generation section 93 under the control of the control section 94 in the control device 90 (see Fig. 7).

Also in the present embodiment, the first output image 413 based on the first image 412, the second output image 423 based on the second image 422, and the superimposed output image 432 based on the superimposed image 431 are generated, and can be simultaneously or switchably displayed on the display device 70. In particular, as in the example illustrated in Fig. 17, the first output image 413, the second output image 423, and the superimposed output image 432 may be the same images as the first image 412, the second image 422, and the superimposed image 431 of the original image, respectively.

The output images 413, 423, and 432 illustrated in parts (d) to (f) of Fig. 17 include only a first image region 451 to which the image of the common image region is allocated, and do not include a second image region 452 different from the first image region 451. In this case, a contour portion of each of the output images 413, 423, and 432 is a portion indicating a boundary of the first image region 451 to which the image of the common image region is allocated. Therefore, in the output images 413, 423, and 432 of the present embodiment, an "additional image for indicating a boundary 445 of the first image region 451" such as the boundary emphasis image 446 (see parts (c) and (d) of Fig. 12 and the like) in the above-described first embodiment is unnecessary.

As described above, according to the present embodiment, the output images 413, 423, and 432 (see parts (d) to (f) of Fig. 17) including only the first image region 451 can be generated. Since the entire output images 413, 423, and 432 are occupied by the first image region 451 in this manner, when the output images 413, 423, and 432 are displayed on the display device 70, all the images in the first image region 451 can be fully displayed in the display region of the display device 70.

### [Modification]

In the first embodiment and the second embodiment described above, the output image is generated on the basis of the entire second image 421, but the output image may be generated on the basis of a partial range of the second image 421. For example, in a case where the observation target is included only in a partial region of the second image 421, the image generation section 93 may extract a partial range of the second image 421 including the region in which the observation target is included, and perform size adjustment processing on the image of the extracted range to generate the second image 421.

Furthermore, in each of the above-described embodiments, the light source device 10 is connected to the insertion device 20 (see Fig. 1) configured as the endoscope main body, but the light source device 10 may be connected to another arbitrary light emission device.

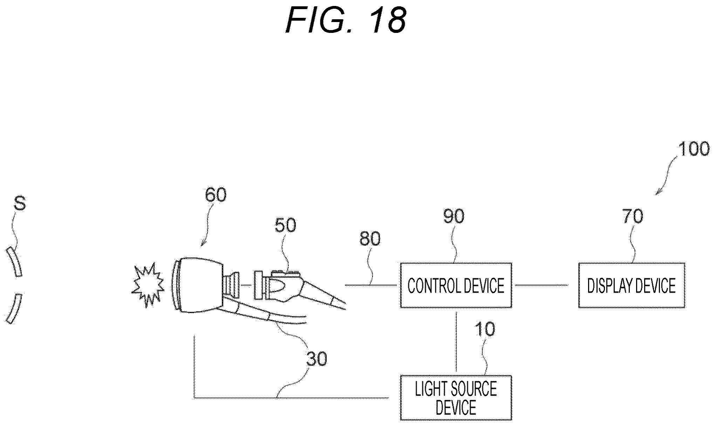

Fig. 18 is a conceptual diagram illustrating an example of the medical observation system 100 configured as an operative field illumination observation device in which a ring light 60 (open field illumination device for observing biological tissue) is connected to a light source device 10.

In the example illustrated in Fig. 18, the light source device 10 and a camera head 50 are connected to the ring light 60. The light emitted by the light source device 10 is transmitted to the ring light 60 via the light guide 30, and is emitted from the ring light 60 toward an observation target S. The ring light 60 is different from the above-described insertion device 20 (see Fig. 1) that is intended to emit light inside the observation target S and irradiate the observation target S with the light in that the ring light 60 is used to emit light outside the observation target S.

In each of the above-described embodiments, instead of the insertion device 20, the ring light 60 of this example may be connected to the light source device 10 and the camera head 50, and the captured image (first image and second image) of the observation target S may be acquired and the output image based on the captured image may be acquired. In this case, observation light from the observation target S enters the camera head 50 through the ring light 60, and a captured image of the observation target S is acquired by the camera head 50 (particularly imaging device 522 of imaging section 52). Then, a control device 90 generates an output image on the basis of the captured image acquired in this manner, and the output image is displayed on a display device 70.

Note that the light emitting device that is connected to the light source device 10 and emits light does not need to be connected to the camera head 50, and may be provided separately from the camera head 50 (particularly imaging section 52).

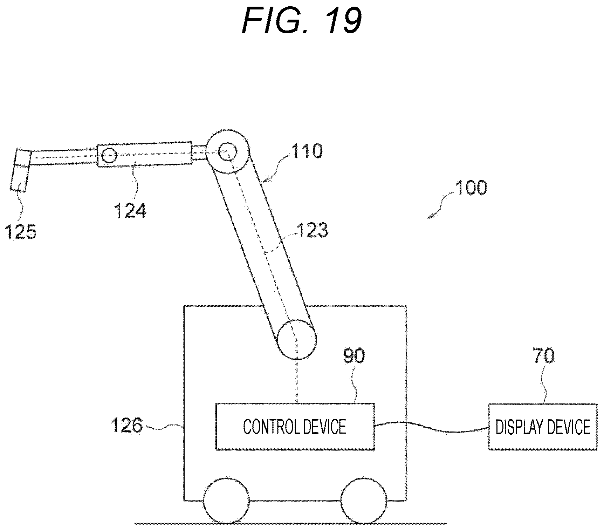

Fig. 19 is a diagram illustrating an example of the medical observation system 100 configured as a microscope system. The medical observation system 100 illustrated in Fig. 19 is a surgical microscope system having a function of enlarging and capturing a visual field area of an observation target and displaying an output image generated on the basis of a captured image.

The medical observation system 100 (surgical microscope system) of this example includes a microscope device 110 that captures an image of an observation target and a display device 70 that displays an image captured by the microscope device 110. The display device 70 illustrated in Fig. 19 is provided separately from the microscope device 110, but may be provided integrally with the microscope device 110.

The microscope device 110 includes a microscope unit 125 that enlarges and images a minute part of the observation target, a support unit 124 having an arm that rotatably supports the microscope unit 125, and a base unit 126 that rotatably supports the support unit 124 and is movable on a floor surface. The base unit 126 includes a control device 90 that controls the operation of the medical observation system 100. The control device 90 is connected to the microscope unit 125 via a transmission cable 123. The base unit 126 may have a configuration fixed to a ceiling, a wall surface, or the like.

The microscope unit 125 includes the above-described imaging section 52 (see Fig. 7; not illustrated in Fig. 19), an operation unit (not illustrated) such as a switch that receives an input of an operation instruction of the microscope device 110, and a cover glass (not illustrated) for protecting the inside. The user can move the microscope unit 125 while operating the operation unit of the microscope unit 125.

In this example, a captured image of the observation target is acquired by the microscope unit 125. Then, similarly to each of the above-described embodiments, the control device 90 generates an output image from the captured image, and the output image is displayed on a display device 70.

It should be noted that the embodiments and modifications disclosed in the present specification are merely illustrative in all respects and are not to be construed as limiting. The above-described embodiments and modifications can be omitted, replaced, and changed in various modes without departing from the scope and spirit of the appended claims. For example, the above-described embodiments and modifications may be combined in whole or in part, and embodiments other than the above-described embodiments and modifications may be combined with the above-described embodiments or modifications. Furthermore, the effects of the present disclosure described in the present specification are merely examples, and other effects may be provided.

The technical category embodying the above technical idea is not limited. For example, the above-described technical idea may be embodied by a computer program for causing a computer to execute one or a plurality of procedures (steps) included in a method of manufacturing or using the above-described device. In addition, the above-described technical idea may be embodied by a computer-readable non-transitory recording medium in which such a computer program is recorded.

### [Supplementary note]

The present disclosure can also have the following configurations.

### [Item 1]

An image generation device that generates an output image on the basis of a first image that is an image obtained by imaging a first range of an observation target using a first imaging device and a second image that is an image obtained by imaging a second range different from the first range of the observation target using a second imaging device different in type from the first imaging device, in which
the output image is an image obtained by performing processing of replacing or deleting one or more pixel signals among pixel signals of the first image and the second image on the basis of the first range and the second range.

### [Item 2]

The image generation device according to item 1, in which
the type is a physical size, an image size, or a model number of the imaging device.

### [Item 3]

The image generation device according to any one of items 1 and 2, in which
a first output image that is the output image, or
a second output image generated by adding one or more pixel signals to an image generated from at least some of the pixel signals of the second image
is output.

### [Item 4]

The image generation device according to any one of items 1 to 3, in which
one of the first range and the second range is wider than the other.

### [Item 5]

The image generation device according to any one of items 1 to 4, in which
a first image region of the output image is generated on the basis of the first image and the second image.

### [Item 6]

The image generation device according to item 5, in which
the output image includes the first image region and a second image region different from the first image region.

### [Item 7]

The image generation device according to item 6, in which
an image visually distinguishable and identifiable from an image allocated to the first image region is allocated to the second image region.

### [Item 8]

The image generation device according to item 5, in which
the output image includes only the first image region.

### [Item 9]

The image generation device according to any one of items 6 and 7, in which
the output image includes a boundary emphasis image indicating a boundary between the first image region and the second image region.

### [Item 10]

The image generation device according to any one of items 1 to 9, in which
the second range is included in the first range.

### [Item 11]

The image generation device according to any one of items 1 to 10, in which
the output image is generated on the basis of the entire second image.

### [Item 12]

The image generation device according to any one of items 5 to 9, in which
one or both of the first image and the second image are enlarged or reduced such that the number of pixels of a common region that is an image region of a part of the observation target commonly included in the first image and the second image is consistent between the first image and the second image, and
an image allocated to the first image region of the output image is generated on the basis of an image of the common region in the first image and an image of the common region in the second image having the same number of pixels.

### [Item 13]

The image generation device according to any one of items 1 to 12, in which
the image generation device is connectable to one or a plurality of display devices on which the output image is displayed, and
the output image corresponding to each characteristic of the one or a plurality of display devices is generated and output.

### [Item 14]

The image generation device according to any one of items 1 to 13, in which
the first image is acquired by imaging the observation target irradiated with first light in a first wavelength band, and
the second image is acquired by imaging the observation target irradiated with second light in a wavelength band at least partially different from the first wavelength band.

### [Item 15]

The image generation device according to item 14, in which
the first light is visible light,
the second light is excitation light that excites a specific substance so as to emit fluorescence,
the first image includes a captured image based on reflected light of the visible light from the observation target, and
the second image includes a captured image based on the fluorescence from the observation target irradiated with the excitation light.

### [Item 16]

A medical observation system including:
an imaging apparatus including a first imaging device and a second imaging device different in type from the first imaging device; and
an image generation device that generates an output image, in which
the image generation device generates the output image on the basis of a first image that is an image obtained by imaging a first range of an observation target using the first imaging device and a second image that is an image obtained by imaging a second range different from the first range of the observation target using the second imaging device, and
the output image is an image obtained by performing processing of replacing or deleting one or more pixel signals among pixel signals of the first image and the second image on the basis of the first range and the second range.

### [Item 17]

The medical observation system according to item 16, further including a light source device that emits light with which the observation target is irradiated, in which
the imaging apparatus images the observation target irradiated with the light and acquires the first image and the second image.

### [Item 18]

The medical observation system according to any one of items 16 and 17, in which
the type is a physical size, an image size, or a model number of the imaging device.

### [Item 19]

The medical observation system according to any one of items 16 to 18, in which
a first output image that is the output image, or
a second output image generated by adding one or more pixel signals to an image generated from at least some of the pixel signals of the second image
is output.

### [Item 20]

The medical observation system according to any one of items 16 to 19, in which
one of the first range and the second range is wider than the other.

### [Item 21]

The medical observation system according to any one of items 16 to 20, in which
a first image region of the output image is generated on the basis of the first image and the second image.

### [Item 22]

The medical observation system according to item 21, in which
the output image includes the first image region and a second image region different from the first image region.

### [Item 23]

The medical observation system according to item 22, in which
an image visually distinguishable and identifiable from an image allocated to the first image region is allocated to the second image region.

### [Item 24]

The medical observation system according to item 21, in which
the output image includes only the first image region.

### [Item 25]

The medical observation system according to any one of items 22 and 23, in which
the output image includes a boundary emphasis image indicating a boundary between the first image region and the second image region.

### [Item 26]

The medical observation system according to any one of items 16 to 25, in which
the second range is included in the first range.

### [Item 27]

The medical observation system according to any one of items 16 to 26, in which
the output image is generated on the basis of the entire second image.

### [Item 28]

The medical observation system according to any one of items 21 to 25, in which
one or both of the first image and the second image are enlarged or reduced such that the number of pixels of a common region that is a part of the observation target commonly included in the first image and the second image is consistent between the first image and the second image, and
an image allocated to the first image region of the output image is generated on the basis of an image of the common region in the first image and an image of the common region in the second image having the same number of pixels.

### [Item 29]

The medical observation system according to any one of items 16 to 28, in which
the medical observation system is connectable to one or a plurality of display devices on which the output image is displayed, and
the output image corresponding to each characteristic of the one or a plurality of display devices is generated and output.

### [Item 30]

The medical observation system according to any one of items 16 to 29, in which
the first image is acquired by imaging the observation target irradiated with first light in a first wavelength band, and
the second image is acquired by imaging the observation target irradiated with second light in a wavelength band at least partially different from the first wavelength band.

### [Item 31]

The medical observation system according to item 30, in which
the first light is visible light,
the second light is excitation light that excites a specific substance so as to emit fluorescence,
the first image includes a captured image based on reflected light of the visible light from the observation target, and
the second image includes a captured image based on the fluorescence from the observation target irradiated with the excitation light.

### [Item 32]

An image generation method including a step of generating an output image on the basis of a first image that is an image obtained by imaging a first range of an observation target using a first imaging device and a second image that is an image obtained by imaging a second range different from the first range of the observation target using a second imaging device different in type from the first imaging device, in which
the output image is an image obtained by performing processing of replacing or deleting one or more pixel signals among pixel signals of the first image and the second image on the basis of the first range and the second range.

### [Item 33]

The image generation method according to item 32, further including:
a size adjustment processing step of adjusting a size of both or one of the first image and the second image; and
a step of generating the output image on the basis of the first image and the second image after the size adjustment processing step.

### [Item 34]

The image generation method according to item 32, further including:
a step of extracting an image of a range in which the observation target is captured in the first image;
a size adjustment processing step of adjusting a size of both or one of the first image and the second image; and
a step of generating the output image on the basis of the extracted first image and the second image after the size adjustment processing step.

### [Item 35]

The image generation method according to any one of items 32 to 34, in which
the type is a physical size, an image size, or a model number of the imaging device.

### [Item 36]

The image generation method according to any one of items 32 to 35, in which
a first output image that is the output image, or
a second output image generated by adding one or more pixel signals to an image generated from at least some of the pixel signals of the second image
is output.

### [Item 37]

The image generation method according to any one of items 32 to 36, in which
one of the first range and the second range is wider than the other.

### [Item 38]

The image generation method according to any one of items 32 to 37, in which
a first image region of the output image is generated on the basis of the first image and the second image.

### [Item 39]

The image generation method according to item 38, in which
the output image includes the first image region and a second image region different from the first image region.

### [Item 40]

The image generation method according to item 39, in which
an image visually distinguishable and identifiable from an image allocated to the first image region is allocated to the second image region.

### [Item 41]

The image generation method according to item 38, in which
the output image includes only the first image region.

### [Item 42]

The image generation method according to any one of items 39 and 40, in which
the output image includes a boundary emphasis image indicating a boundary between the first image region and the second image region.

### [Item 43]

The image generation method according to any one of items 32 to 42, in which
the second range is included in the first range.

### [Item 44]

The image generation method according to any one of items 32 to 43, in which
the output image is generated on the basis of the entire second image.

### [Item 45]

The image generation method according to any one of items 38 to 42, in which
one or both of the first image and the second image are enlarged or reduced such that the number of pixels of a common region that is a part of the observation target commonly included in the first image and the second image is consistent between the first image and the second image, and
an image allocated to the first image region of the output image is generated on the basis of an image of the common region in the first image and an image of the common region in the second image having the same number of pixels.

### [Item 46]

The image generation method according to any one of items 32 to 45, in which
one or a plurality of connectable display devices on which the output image is displayed are provided, and
the output image corresponding to each characteristic of the one or a plurality of display devices is generated and output.

### [Item 47]

The image generation method according to any one of items 32 to 46, in which
the first image is acquired by imaging the observation target irradiated with first light in a first wavelength band, and
the second image is acquired by imaging the observation target irradiated with second light in a wavelength band at least partially different from the first wavelength band.

### [Item 48]

The image generation method according to item 47, in which
the first light is visible light,
the second light is excitation light that excites a specific substance so as to emit fluorescence,
the first image includes a captured image based on reflected light of the visible light from the observation target, and
the second image includes a captured image based on the fluorescence from the observation target irradiated with the excitation light.

### REFERENCE SIGNS LIST

- 10: Light source device
- 11: First light source
- 12: Second light source
- 15: Optical element
- 20: Insertion device
- 21: Insertion portion
- 21a: Distal end portion
- 22: Optical connection portion
- 23: Imaging connection portion
- 30: Light guide
- 40: Lens optical system
- 41: Mirror optical system
- 50: Camera head
- 51: Lens unit
- 52: Imaging section
- 53: Communication section
- 60: Ring light
- 70: Display device
- 80: Transmission cable
- 90: Control device
- 91: Communication section
- 92: Memory
- 93: Image generation section
- 94: Control section
- 95: Input section
- 96: Output section
- 97: Storage section
- 100: Medical observation system
- 110: Microscope device
- 123: Transmission cable
- 124: Support unit
- 125: Microscope unit
- 126: Base unit
- 410: Imaging region
- 410A: Common light receiving region
- 410B: Non-common light receiving region
- 411: First image
- 411A: Common image region
- 411B: Non-common image region
- 412: First image
- 413: First output image
- 420: Imaging region
- 421: Second image
- 422: Second image
- 423: Second output image
- 431: Superimposed image
- 431A: Superimposing region
- 431B: Non-superimposing region
- 432: Superimposed output image
- 445: Boundary
- 446: Boundary emphasis image
- 451: First image region
- 452: Second image region
- 521: Light incident portion
- 522: Imaging device
- 522a: First imaging device
- 522b: Second imaging device
- 522c: Third imaging device
- 523: Signal processor
- 930: Memory controller
- 931: Image processor
- 934: Superimposed image generator
- 935: Display control portion
- A: Organ
- B1: Fluorescent site
- B2: Fluorescent site
- F: Boundary between superimposed region and non-superimposed region
- Lf: Observation light
- Lf1: First light flux
- Lf2: Second light flux
- P1: Pixel
- P2: Pixel
- S: Observation target

## Claims

1. An image generation device that generates an output image on a basis of a first image that is an image obtained by imaging a first range of an observation target using a first imaging device and a second image that is an image obtained by imaging a second range different from the first range of the observation target using a second imaging device different in type from the first imaging device, wherein
the output image is an image obtained by performing processing of replacing or deleting one or more pixel signals among pixel signals of the first image and the second image on a basis of the first range and the second range.

2. The image generation device according to claim 1, wherein
the type is a physical size, an image size, or a model number of the imaging device.

3. The image generation device according to claim 1, wherein
a first output image that is the output image, or
a second output image generated by adding one or more pixel signals to an image generated from at least some of the pixel signals of the second image
is output.

4. The image generation device according to claim 1, wherein
one of the first range and the second range is wider than the other.

5. The image generation device according to claim 1, wherein
a first image region of the output image is generated on a basis of the first image and the second image.

6. The image generation device according to claim 5, wherein
the output image includes the first image region and a second image region different from the first image region.

7. The image generation device according to claim 6, wherein
an image visually distinguishable and identifiable from an image allocated to the first image region is allocated to the second image region.

8. The image generation device according to claim 5, wherein
the output image includes only the first image region.

9. The image generation device according to claim 6, wherein
the output image includes a boundary emphasis image indicating a boundary between the first image region and the second image region.

10. The image generation device according to claim 1, wherein
the second range is included in the first range.

11. The image generation device according to claim 1, wherein
the output image is generated on a basis of the entire second image.

12. The image generation device according to claim 5, wherein
one or both of the first image and the second image are enlarged or reduced such that the number of pixels of a common image region that is an image region of a part of the observation target commonly included in the first image and the second image is consistent between the first image and the second image, and
an image allocated to the first image region of the output image is generated on a basis of an image of the common image region in the first image and an image of the common image region in the second image having the same number of pixels.

13. The image generation device according to claim 1, wherein
the image generation device is connectable to one or a plurality of display devices on which the output image is displayed, and
the output image corresponding to each characteristic of the one or a plurality of display devices is generated and output.

14. The image generation device according to claim 1, wherein
the first image is acquired by imaging the observation target irradiated with first light in a first wavelength band, and
the second image is acquired by imaging the observation target irradiated with second light in a wavelength band at least partially different from the first wavelength band.

15. The image generation device according to claim 14, wherein
the first light is visible light,
the second light is excitation light that excites a specific substance so as to emit fluorescence,
the first image includes a captured image based on reflected light of the visible light from the observation target, and
the second image includes a captured image based on the fluorescence from the observation target irradiated with the excitation light.

16. A medical observation system comprising
an imaging apparatus including a first imaging device and a second imaging device different in type from the first imaging device; and
an image generation device that generates an output image, wherein
the image generation device generates the output image on a basis of a first image that is an image obtained by imaging a first range of an observation target using the first imaging device and a second image that is an image obtained by imaging a second range different from the first range of the observation target using the second imaging device, and
the output image is an image obtained by performing processing of replacing or deleting one or more pixel signals among pixel signals of the first image and the second image on a basis of the first range and the second range.

17. The medical observation system according to claim 16, further comprising a light source device that emits light with which the observation target is irradiated, wherein
the imaging apparatus images the observation target irradiated with the light and acquires the first image and the second image.

18. An image generation method comprising a step of generating an output image on a basis of a first image that is an image obtained by imaging a first range of an observation target using a first imaging device and a second image that is an image obtained by imaging a second range different from the first range of the observation target using a second imaging device different in type from the first imaging device, wherein
the output image is an image obtained by performing processing of replacing or deleting one or more pixel signals among pixel signals of the first image and the second image on a basis of the first range and the second range.

19. The image generation method according to claim 18, further comprising:
a size adjustment processing step of adjusting a size of both or one of the first image and the second image; and
a step of generating the output image on a basis of the first image and the second image after the size adjustment processing step.

20. The image generation method according to claim 18, further comprising:
a step of extracting an image of a range in which the observation target is captured in the first image;
a size adjustment processing step of adjusting a size of both or one of the first image and the second image; and
a step of generating the output image on a basis of the extracted first image and the second image after the size adjustment processing step.
